# EUROPÄISCHE PATENTSCHRIFT

(11) **EP 3 046 472 B1**
(45) Veröffentlichungstag und Bekanntmachung des Hinweises auf die Patenterteilung: **02.08.2017**
(21) Anmeldenummer: 14796659.2
(22) Anmeldetag: 15.09.2014
(51) Int. Cl.: A61B 5/15, A61B 5/153, A61B 5/154

(54) **ABNAHMEEINHEIT FÜR KÖRPERFLÜSSIGKEITEN, INSBESONDERE BLUT**
REMOVAL UNIT FOR BODILY FLUIDS, IN PARTICULAR BLOOD
UNITÉ DE PRÉLÈVEMENT POUR DES LIQUIDES CORPORELS, EN PARTICULIER DU SANG

(30) Priorität: 17.09.2013 AT 505882013
(43) Veröffentlichungstag der Anmeldung: 27.07.2016
(73) Patentinhaber: Greiner Bio-One GmbH, 4550 Kremsmünster (AT)
(72) Erfinder: EBETSBERGER, Franz, A-4550 Kremsmünster (AT); STRASSER, Gerhard, A-4550 Kremsmünster (AT)
(74) Vertreter: Burger, Hannes
(86) Internationale Anmeldenummer: PCT/AT2014/050204
(87) Internationale Veröffentlichungsnummer: WO 2015/039153

(56) Entgegenhaltungen:
- EP-A1- 0 478 459
- EP-A1- 1 602 329
- EP-A2- 0 146 691
- EP-B1- 0 619 096
- WO-A1-2011/162772
- US-A- 3 585 984

## Beschreibung

Die Erfindung betrifft eine medizinische Abnahmeeinheit für Körperflüssigkeiten, insbesondere Blut, wie diese im Anspruch 1 beschrieben ist.

Eine gattungsgemäße Blutprobenentnahme-Nadelanordnung ist aus der EP 0 619 096 B1 bzw. der US 5,520,193 A bekannt geworden. Diese umfasst eine Nadelkanüle mit einem ersten Ende, das für die Einführung in eine Vene eines Blutspenders geeignet ist. Ein zweites, schräg geschnittenes Ende der Nadelkanüle ist für das Einführen in einen unter Unterdruck stehenden Blutsammelcontainer geeignet, der dichtend mit der Nadelkanüle verbindbar ist. Eine Nadelkanülen-Trägervorrichtung ist in einem Mittelabschnitt der Nadelkanüle angebracht, sodass sich das erste Ende der Nadelkanüle nach außen erstreckt und flüssigkeitsdicht mit einer ersten Seite der Trägervorrichtung verbunden ist. Das zweite Ende der Nadelkanüle erstreckt sich nach außen in dazu entgegengesetzter Richtung. Weiters ist auf der Trägervorrichtung über dem zweiten Ende der Nadelkanüle eine elastisch federnde Kappe angebracht. Das Material zur Bildung der Trägervorrichtung ist aus einem durchsichtigen oder transparenten Material gebildet. In der Trägervorrichtung ist ein radialer Durchgang als Indikationskammer angeordnet, welche über einen sich in Axialrichtung erstreckenden Durchgang mit der von der federnden Kappe umschlossenen Aufnahmekammer in Strömungsverbindung steht. Der radiale Durchgang ist gegenüber der äußeren Umgebung mit einem luftdurchlässigen, jedoch flüssigkeitsabweisenden Pfropfen verschlossen.

Eine andere Blutentnahmevorrichtung ist aus der DE 32 29 783 C1 bekannt geworden und umfasst eine doppelendige Kanüle, an welcher ein Kanülenhalter befestigt ist. Am proximalen Ende des Kanülenhalters ist ein rohrförmiger Ansatz angeordnet, an welchem eine flexible Hülle gehalten ist. Weiters ist auf dem rohrförmigen Ansatz noch ein poröser Ring vorgesehen. An der Außenseite des rohrförmigen Ansatzes sind zusätzlich noch mehrere Längsnuten ausgebildet, die sich zumindest bis zum porösen Ring erstrecken. Der von der Hülle umschlossene Aufnahmeraum steht über die Längsnuten sowie den porösen Ring mit der äußeren Umgebung in Strömungsverbindung. Der poröse Ring ermöglicht einen Luftaustritt aus der Hülle an die Umgebung, blockiert jedoch einen Blutdurchtritt. Zur Anzeige des Ansticherfolgs ist die Hülle aus transparentem Latex gebildet.

Eine weitere Anzeigevorrichtung für den Ansticherfolg bei einer Blutabnahmevorrichtung ist aus der US 6,905,483 B2 bekannt geworden. Dabei umfasst die medizinische Vorrichtung einen Nadelträger, in welchem eine Indikationskammer angeordnet ist, welche mit einem Entlüftungsstopfen gegenüber der äußeren Umgebung abgeschlossen ist. Der Entlüftungsstopfen ist aus einem Material gebildet, welches ein Hindurchströmen von Luft ermöglicht, jedoch ein Hindurchströmen von Flüssigkeit verhindert. Dazu kann der Verschlussstopfen eine Membran aufweisen, welche eine Vielzahl von mikroskopischen Öffnungen oder Poren hat. Eine Kanüle mit proximalem sowie distalem Ende ragt beidseits aus dem Nadelträger heraus und ist mit diesem verbunden. Ein Durchgangskanal der Kanüle steht in Strömungsverbindung mit der Indikationskammer. Eine schlauchförmige Hülle umschließt den proximalen Kanülenabschnitt unter Ausbildung eines Aufnahmeraums. Das bei einem Anstich vom distalen Ende der Kanüle einströmende Blut verdrängt die Luft aus der Kanüle und drückt diese über die Indikationskammer und den luftdurchlässigen Verschlussstopfen hin zur äußeren Umgebung.

Weitere Blutabnahme-Nadelanordnungen mit einem Filterelement, welches ein Hindurchströmen von Luft ermöglicht, jedoch ein Hindurchströmen von Blut unterbindet, sind beispielsweise aus der EP 0 451 040 A1 sowie der JP 36 74946 B2 bekannt geworden.

Aus der EP 0 396 537 B1 bzw. der DE 37 85 363 T2 ist eine Vorrichtung zur Strömungsanzeige bekannt geworden, welche in erster Linie für die Unterstützung und genauen Platzierung eines Kathederaufbaus in einem Blutgefäß dient. Bei der Verwendung im Umfang der sauberen Platzierung des Kathederaufbaus durchdringt zunächst eine geschärfte Spitze der unterstützenden Nadel oder Kanüle, auf welcher die Kathederstruktur befestigt ist, das dafür vorgesehene Blutgefäß. Wenn die geschärfte Spitze ordnungsgemäß positioniert ist, fließt Blut kontinuierlich durch die hohle Unterstützungsnadel von der Spitze zu ihrem proximalen Ende, welches fest an einer Haltebasis angebracht ist. Um während des Setzens des Kathederaufbaus dem medizinischen Personal die ordnungsgemäße Lage des Katheders innerhalb des Blutgefäßes anzuzeigen, weist die Vorrichtung zur Strömungsanzeige einen Basiskörper mit einer länglichen, im Wesentlichen zylindrischen Konfiguration auf, an dessen distalem Ende sich eine Nadel nach außen erstreckt. Die Nadel weist einen hohlen Innenabschnitt auf, der sich entlang ihrer Länge von der Spitze zu dessen proximalen Ende erstreckt. Das proximale Ende ist an der Innenseite des Basiskörpers befestigt, wobei das Blut längs der Nadel in die Basis hineinströmt, wenn die Spitze in dem Blutgefäß platziert ist. Weiters ist an der Außenseite des Basiskörpers eine wenigstens teilweise transparent ausgebildete Umhüllungseinrichtung angeordnet. An der Außenseite des Basiskörpers ist weiters ein Fließkanal vorgesehen, der in Form einer Nutenstruktur mit einer Vielzahl von Nutensegmenten gebildet ist, welche untereinander durch versetzt zueinander angeordnete Anschlussöffnungen in Fluidkommunikation stehen. Am Ende des Fließkanals ist eine Entlüftungsöffnung vorgesehen, durch welche die im Fließkanal enthaltene Luft an die Umgebung ausströmen kann. Durch diesen verschlungen ausgebildeten Fließkanal kann während des Setzens der Kathederanordnung stets dessen ordnungsgemäße Positionierung innerhalb des Blutgefäßes kontrolliert werden. Sobald die Kathederanordnung ordnungsgemäß gesetzt ist, wird die Vorrichtung zur Strömungsanzeige entfernt und die Kathederanordnung verbleibt am Patienten.

Der vorliegenden Erfindung liegt die Aufgabe zugrunde, eine medizinische Abnahmeeinheit bzw. Einsticheinheit für die Abnahme von Körperflüssigkeiten, insbesondere Blut, zu schaffen, bei welcher eine Anzeige des Ansticherfolgs selbsttätig erfolgt und dabei kostengünstig ohne der Verwendung eines Filterelements herzustellen ist sowie eine hohe Betriebssicherheit aufweist.

Diese Aufgabe der Erfindung wird durch die Merkmale des Anspruches 1 gelöst.

Der sich durch die Merkmale des Anspruches 1 ergebende Vorteil liegt darin, dass es bei der erfindungsgemäßen Abnahmeeinheit möglich ist, im Zuge des Anstichvorgangs die im Inneren der Kanüle sowie der schlauchförmigen Hülle vorhandene Luft aus der Abnahmeeinheit abströmen kann. Dies erfolgt durch den Abströmkanal, welcher mehrere in Strömungsrichtung hintereinander angeordnete Verbindungskanäle umfasst, bei denen der Strömungsquerschnitt derart gewählt ist, dass zwar ein Hindurchtreten von Luft ermöglicht, jedoch ein Hindurchtreten von Blut verhindert ist. Durch das zusätzliche Vorsehen von mehreren Kammern zwischen den im Abströmkanal angeordneten Verbindungskanälen kann so eine Art Labyrinthdichtung geschaffen werden, bei der die Dichtwirkung auf rein mechanischer Basis erzielt wird. Durch das labyrinthartige hintereinander Anordnen der voneinander getrennten Kammern sowie Verbindungskanälen, können so innerhalb des Abströmkanals für die daraus abzuleitende Luftmenge unterschiedliche Strömungsgeschwindigkeiten zueinander erzielt werden. Durch das Vorsehen der reinen mechanischen Entlüftungsvorrichtung kann so auf den Einsatz teurer Filterelemente verzichtet werden und trotzdem ein Abströmen der innerhalb der Kanüle vorhandenen Luft im Zuge des Anstichvorgangs erreicht werden. So kann in weiterer Folge nicht nur der Ansticherfolg optisch dargestellt werden, sondern es wird auch ein Austritt der eintretenden Körperflüssigkeit, insbesondere von Blut, durch die Entlüftungsvorrichtung hindurch verhindert. Sollte bei einem mehrfachen Abnahmevorgang und dem damit verbundenen, erhöhten Druckaufbau innerhalb der Hülle die dort bevorratete Körperflüssigkeitsmenge, insbesondere das Blut, doch durch einen der Verbindungskanäle in die darauffolgende Kammer eintreten, so dient diese als zusätzlicher Speicherraum zur Aufnahme der Körperflüssigkeit. Damit kann ein Austreten derselben am Ende des Abströmkanals gesichert verhindert werden.

Vorteilhaft ist auch eine weitere Ausführungsform nach Anspruch 2, da so durch die gegenseitige Versetzung von unmittelbar in Ausströmrichtung hintereinander angeordneten Verbindungskanälen in Umfangsrichtung zueinander eine Umlenkung des Luftstromes innerhalb der dazwischen angeordneten Kammer erfolgt. Darüber hinaus kann so aber durch die bewusste Versetzung bei einem möglichen Einströmen der Körperflüssigkeit, insbesondere von Blut, ein Weiterströmen von Luft ermöglicht werden. Ein unmittelbares Weiterströmen der eingetretenen Körperflüssigkeit aus der Kammer kann bis zu einem gewissen Füllungsgrad derselben in weiterer Folge gesichert verhindert werden.

Vorteilhaft ist weiters eine Ausbildung nach Anspruch 3, da so ein durchgängiger Verbindungskanal zur Bildung des Abströmkanals vermieden wird. Dadurch kann nicht nur eine Labyrinthdichtung geschaffen werden, sondern es kann damit ein ungewollter Austritt der Körperflüssigkeit aus dem Abströmkanal an die Umgebung verhindert werden. Dadurch wird auf einfache Art und Weise eine Abströmmöglichkeit für die innerhalb der Abnahmeeinheit vorhandene Luft geschaffen und trotzdem eine hohe Betriebssicherheit erzielt.

Durch die Ausbildung nach Anspruch 4 ist es möglich, den Nadelträger aufgrund seiner körperlichen Aufteilung in den Basiskörper sowie den darin einzusetzenden und aufgenommenen Einsatzteil eine einfach herzustellende Baueinheit zu schaffen. Damit kann, ohne zusätzlicher, komplizierter Fertigungsvorgänge, auf einfache Art und Weise ein Nadelträger, beispielsweise aus einem Kunststoffmaterial, gefertigt werden, welcher in einem Spritzgusswerkzeug mit hoher Präzision hergestellt werden kann. Damit kann ein Ausbilden der Kammern sowie der Verbindungskanäle entweder direkt im Zuge des Herstellvorganges oder aber auch durch einen einfachen Nachbearbeitungsschritt durchgeführt werden. Die Ausbildung der Kammern kann entweder durch entsprechende, geometrische Abstimmung der beiden Bauteile zueinander oder aber auch durch entsprechende Formenwahl realisiert werden.

Nach einer anderen Ausführungsvariante gemäß Anspruch 5 wird so eine einfach herzustellende Baueinheit geschaffen wird, welche aus zusammenfügbaren Bauteilen besteht und sich dadurch eine kostengünstige Herstellung erzielen lässt.

Vorteilhaft ist auch eine Weiterbildung nach Anspruch 6, da dadurch ein einfach herzustellender Basisteil bzw. Basiskörper geschaffen werden kann, in welchen ein vollständig ausgebildeter Einsatzteil lediglich eingesetzt und daran feststehend gehalten werden muss. Weiters kann dadurch aber auch die Größe und Lage der einzelnen Kammern exakt festgelegt werden.

Gemäß einer Ausbildung, wie im Anspruch 7 beschrieben, können so einfach Ringräume geschaffen werden, die einfach einzuformen sind und durch entsprechende Anordnung und Ausbildung der Verbindungskanäle ein Abströmkanal geschaffen werden, welcher ein ungehindertes Abströmen von Luft ermöglicht, jedoch ein Hindurchströmen von Blut bis hin zum Austritt an die äußere Umgebung sicher verhindert.

Vorteilhaft ist auch eine Weiterbildung nach Anspruch 8, da so in Art eines Gewindes der Abströmkanal im Einsatzteil ausgebildet werden kann, der lediglich durch mehrere in Längserstreckung des Abströmkanals angeordnete Trennstege in die einzelnen Kammern unterteilt ist. Im Bereich der Trennstege sind jeweils die entsprechenden Verbindungskanäle angeordnet bzw. ausgebildet, um so wiederum in diesen Abschnitten nur ein Hindurchströmen von Luft zu ermöglichen. Durch diesen spiralförmigen Längsverlauf können so einfach mehrere Kammern hintereinander ausgebildet werden, wobei dies von der Anzahl sowie Anordnung der Trennstege abhängig ist.

Bei der Ausgestaltung nach Anspruch 9 oder 10 ist von Vorteil, dass so mit zunehmender Strömungslänge innerhalb der Abnahmeeinheit die Größe und damit verbunden das Aufnahmevolumen der einzelnen Kammern in Richtung der Ausströmrichtung vergrößert werden kann. So kann in weiterer Folge auch noch die Sicherheit der gesamten Abnahmeeinheit durch das größer werdende und zur Verfügung stehende Aufnahmevolumen der einzelnen Kammern erhöht werden.

Vorteilhaft ist auch eine Ausbildung nach Anspruch 11, da so ein relativ großes Aufnahmevolumen innerhalb der einzelnen Kammern geschaffen werden kann. Darüber hinaus wird aber so auch eine in Umfangsrichtung beidseitige Strömung innerhalb des jeweiligen Kanals zwischen den Verbindungskanälen ermöglicht.

Durch die Ausbildung nach Anspruch 12 kann eine sichere, gasdichte Halterung der Kanüle innerhalb des Nadelträgers geschaffen werden. Diese Verbindung könnte entweder im Zuge des Herstellvorganges des Nadelträgers, insbesondere dessen Basiskörper, geschaffen werden oder aber auch mit einem nachträglichen Verbindungsvorgang, wie beispielsweise einem Klebevorgang, erfolgen.

Gemäß einer Ausbildung, wie im Anspruch 13 beschrieben, kann so im unmittelbaren Anschluss an die durch die Hülle gebildete Aufnahmekammer ein gerichtetes Durchströmen der abzuströmenden Luft im Innersten des Einsatzteils zwischen diesem und der Kanüle erfolgen. Dadurch kann ein geradliniges Abströmen der Luft ohne große Umlenkungen erfolgen, wodurch die selbsttätige Abströmung der Luft doch durch den relativ geringen Blutdruck selbsttätig erfolgen kann. Damit werden Strömungshindernisse, wie diese bei der Umlenkung des Luftstromes sonst auftreten können, vermieden.

Dabei erweist sich eine Ausgestaltung nach Anspruch 14 vorteilhaft, weil dadurch bereits im Zuge des Anstichvorgangs die Körperflüssigkeit, insbesondere das Blut, bis in den dafür vorgesehenen Abschnitt des Abströmkanals selbsttätig einströmen kann und so den Benutzer ohne zusätzlicher Hilfsmittel die ordnungsgemäße Positionierung der Kanüle innerhalb des Körpers des Patienten optisch sichtbar gemacht werden kann.

Nach einer vorteilhaften Weiterbildung gemäß Anspruch 15 wird so bereits am Beginn des Abströmkanals innerhalb des Nadelträgers die optische Kontrolle des Ansticherfolgs erfolgen kann. Die Anordnung der Entlüftungsvorrichtung kann im Anschluss an die Indikationskammer erfolgen, um so zumindest beim Vorsehen der Indikationskammern ein gesichertes Einströmen der Körperflüssigkeit bis dorthin zu ermöglichen. Dieses Einströmen in die Indikationskammer erfolgt selbsttätig im Zuge des ordnungsgemäßen Anstichvorgangs, wobei bis zur erfolgen Anzeige ein Abströmen der Luft aus der Indikationskammer durch den Abströmkanal und die anschließende Entlüftungsvorrichtung hindurch erfolgt.

Von Vorteil ist aber auch eine Ausbildung nach Anspruch 16, da so auf das Vorsehen einer eigenen Indikationskammer verzichtet werden kann und bereits unmittelbar anschließend an den Austritt der Körperflüssigkeit im Bereich des proximalen Endes der Kanüle die optische Anzeige bereits im Bereich der Hülle einfach erfolgen kann.

Gemäß Anspruch 17 wird so bei Vorsehen einer Indikationskammer auch in diesem Bereich die optische Kontrolle des ordnungsgemäßen Anstichvorgangs möglich.

Bei der Ausbildung gemäß Anspruch 18 wird erreicht, dass so eine einteilig ausgebildete Kanüle verwendet werden kann, bei welchem zwischen dem distalen und dem proximalen Ende ein durchgängiger, ununterbrochener Abnahmekanal mit einem gleichen Strömungsquerschnitt als Verbindung zwischen dem Patienten und der Blutabnahmevorrichtung zur Verfügung steht. Durch diesen durchgängigen, gleichmäßigen Strömungsquerschnitt können negative Beeinflussungen der Körperflüssigkeiten, insbesondere des Blutes, für einen nachfolgenden, einwandfreien Analysevorgang erzielt werden.

Möglich ist dabei auch eine Ausbildung nach Anspruch 19 da so die Abnahmeeinheit als Baugruppe vorgefertigt werden kann und je nach Bedarf in ein dafür vorgesehenes Handhabungsgerät eingesetzt und damit verbunden werden kann. Damit kann eine einfache, flexible Anwendung der Abnahmeeinheit für unterschiedlichste Einsatzbedingungen geschaffen werden.

Schließlich ist aber auch eine Ausbildung, wie im Anspruch 20 beschrieben möglich, da dadurch bereits eine vorgefertigte Baueinheit zur Verfügung gestellt werden kann, bei welcher ein hoher Vorfertigungs- und Montagegrad erzielbar ist. Damit kann ohne einen weiteren Montage- bzw. Fügevorgang es dem Benutzer ermöglicht werden, die Abnahmeeinheit unmittelbar einsetzen zu können.

Zum besseren Verständnis der Erfindung wird diese anhand der nachfolgenden Figuren näher erläutert.

Es zeigen jeweils in stark vereinfachter, schematischer Darstellung:
- Fig. 1: eine erste Ausführungsform einer Abnahmeeinheit mit einem zusätzlich daran angeordneten Handhabungselement, in schaubildlicher Darstellung;
- Fig. 2: die Abnahmeeinheit mit dem Handhabungselement nach Fig. 1, im Axialschnitt;
- Fig. 3: die Abnahmeeinheit mit dem Handhabungselement nach den Fig. 1 und 2, in voneinander distanzierter Stellung der einzelnen Bauteile, im Axialschnitt sowie schaubildlicher Darstellung;
- Fig. 4: den Einsatzteil des Nadelträgers der Abnahmeeinheit nach den Fig. 1 bis 3, in schaubildlicher Darstellung;
- Fig. 5: eine zweite Ausführungsform einer Abnahmeeinheit, in schaubildlicher Darstellung;
- Fig. 6: die Abnahmeeinheit nach Fig. 5, im Axialschnitt;
- Fig. 7: die Abnahmeeinheit nach den Fig. 5 und 6, in voneinander distanzierter Stellung der einzelnen Bauteile, im Axialschnitt sowie schaubildlicher Darstellung;
- Fig. 8: den Einsatzteil des Nadelträgers der Abnahmeeinheit nach den Fig. 5 bis 7, in schaubildlicher Darstellung;
- Fig. 9: eine dritte Ausführungsform einer Abnahmeeinheit, in schaubildlicher Darstellung;
- Fig. 10: die Abnahmeeinheit nach Fig. 9, in einer anderen schaubildlichen Ansicht;
- Fig. 11: die Abnahmeeinheit nach den Fig. 9 und 10, in Ansicht gemäß Pfeil XI in Fig. 10;
- Fig. 12: die Abnahmeeinheit nach den Fig. 9 bis 11, im Axialschnitt gemäß den Linien XII-XII in Fig. 10.

Einführend sei festgehalten, dass in den unterschiedlich beschriebenen Ausführungsformen gleiche Teile mit gleichen Bezugszeichen bzw. gleichen Bauteilbezeichnungen versehen werden, wobei die in der gesamten Beschreibung enthaltenen Offenbarungen sinngemäß auf gleiche Teile mit gleichen Bezugszeichen bzw. gleichen Bauteilbezeichnungen übertragen werden können. Auch sind die in der Beschreibung gewählten Lageangaben, wie z.B. oben, unten, seitlich usw. auf die unmittelbar beschriebene sowie dargestellte Figur bezogen und sind diese Lageangaben bei einer Lageänderung sinngemäß auf die neue Lage zu übertragen.

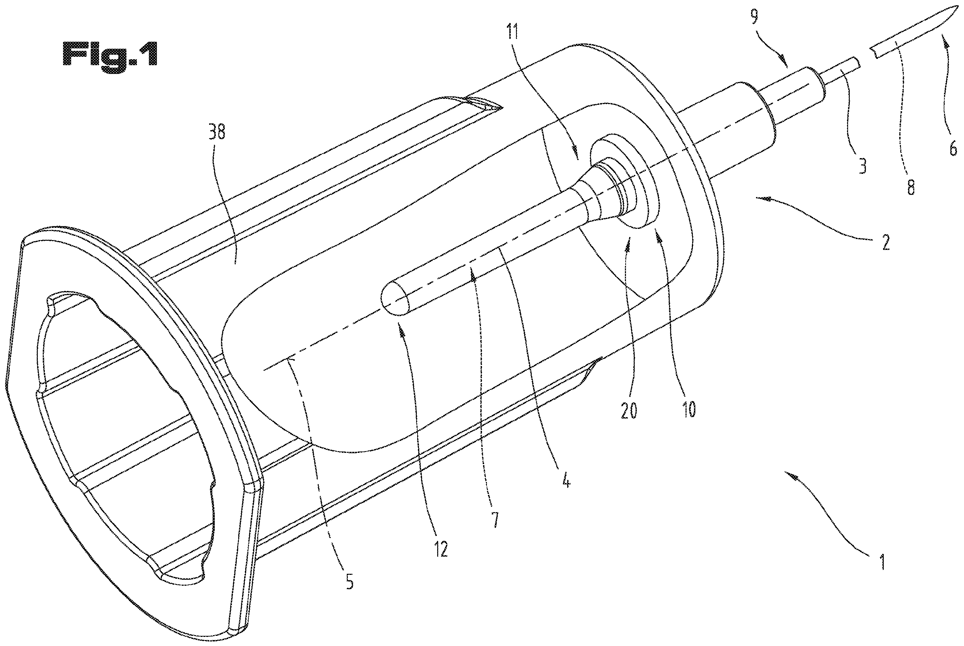

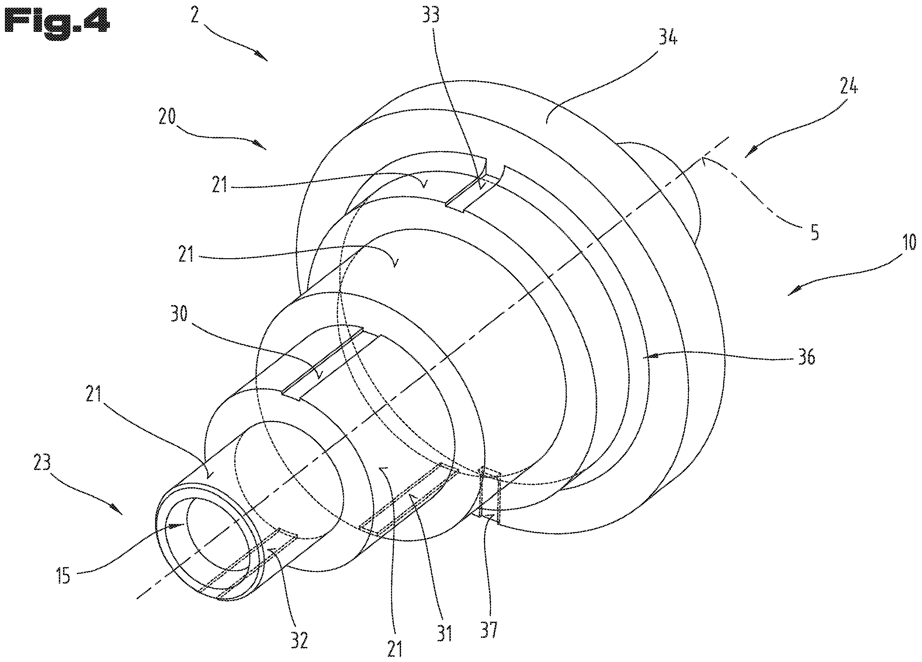

In den Fig. 1 bis 4 ist eine erste mögliche Ausführungsform einer medizinischen Abnahmeeinheit 1 gezeigt, welche zumindest eine Kanüle 3, sowie eine schlauchförmig ausgebildete, elastisch verformbar sowie durchstechbare Hülle 4 umfasst.

Weiters sei hier allgemein erwähnt, dass in der vorliegenden Anmeldung mit der Bezeichnung "distal" steht jener Teil der Abnahmeeinheit 1 bezeichnet wird, welche bei der bestimmungsgemäßen Verwendung durch einen Benutzer (medizinisches Personal) von diesem abgewendet und somit beispielsweise einem Körper eines Patienten zugewendet ist. Als "proximal" wird stets jener Teil der Abnahmeeinheit 1 bezeichnet, welche bei der bestimmungsgemäßen Verwendung durch einen Benutzer (medizinisches Personal) diesem zugewendet und somit zum Beispiel von einem Körper des Patienten abgewendet ist. Gleiches gilt aber auch für die Richtungsbezeichnung einzelner die Abnahmeeinheit 1 bildenden Bauteile, welche jeweils ihrerseits wiederum in Richtung einer Längsachse 5 gesehen distale sowie proximale Enden bzw. Seiten aufweisen können, welche in weiterer Folge mit der entsprechenden Bauteilbezeichnung näher bezeichnet werden.

Die Abnahmeeinheit 1, umfassend den Nadelträger 2 mit der daran gehaltenen Kanüle 3, kann auch als Einsticheinheit bezeichnet werden, welche für sich alleine Verwendung finden kann oder aber auch an einer später noch näher beschriebenen Handhabungsvorrichtung angebracht oder teilweise damit ausgebildet werden kann.

Die hier beschriebenen Abnahmeeinheiten 1 werden im medizinischen Bereich überwiegend für die Abnahme von Körperflüssigkeiten, insbesondere Blut eingesetzt. Dazu wird ein distaler Kanülenabschnitt 6 in bekannter Weise von einem Benutzer, wie beispielsweise einem Arzt oder einem medizinischen Personal in den Körper des Patienten in eine dafür vorgesehene Vene oder Arterie eingestochen. Für den nachfolgenden Abnahmevorgang werden zumeist evakuierte Blutprobenentnahmeröhrchen mit einem proximalen Kanülenabschnitt 7 der Kanüle 3 nacheinander durch einen Einstichvorgang in Strömungsverbindung gebracht. Derartige Blutprobenentnahmeröhrchen, welche auch als Sammelbehälter bezeichnet werden können, weisen einen durchstechbaren, selbstverschließenden Verschlusskörper, insbesondere einen Dichtstopfen auf, welcher zusätzlich noch zum leichteren Hantieren desselben mit einer Kappe versehen sein kann. Zur Herstellung der Strömungsverbindung wird der Verschlusskörper bzw. der Dichtstopfen des Blutprobenentnahmeröhrchens vom proximalen Kanülenabschnitt 7 durchstochen und so eine Strömungsverbindung zwischen der Vene oder der Arterie des Patienten und dem Innenraum des als Sammelbehälter ausgebildeten Blutentnahmeröhrchens hergestellt. Beim Anstichvorgang wird die den proximalen Kanülenabschnitt 7 abdeckende Hülle 4 von der Kanüle 3 durchstochen und bei weiterem Einschieben die Hülle 4 in Richtung auf den Nadelträger 2 geschoben und dabei zusammengefaltet.

Diese so hergestellte Strömungsverbindung zwischen dem distalen Kanülenabschnitt 6 und dem proximalen Kanülenabschnitt 7 kann auch als sogenannter Abnahmekanal 8 bezeichnet werden, welcher den distalen Kanülenabschnitt 6 mit dem proximalen Kanülenabschnitt 7 für ein Durchströmen verbindet. Bevorzugt wird der Abnahmekanal 8 durchgängig zwischen dem distalen Kanülenabschnitt 6 und dem proximalen Kanülenabschnitt 7 innerhalb der Kanüle 3 ausgebildet. Bei dieser Ausführungsform kann auch von einer doppelendigen, durchgängigen Kanüle 3 gesprochen werden.

Der Nadelträger 2 kann durch eine Baugruppe aus mehreren Bauteilen gebildet sein, wobei der Nadelträger 2 ein distales Nadelträgerende 9 sowie ein proximales Nadelträgerende 10 aufweist.

Die schlauchförmig ausgebildete Hülle 4 weist ihrerseits ein offenes distales Hüllenende 11 sowie am gegenüberliegenden Ende ein verschlossenes proximales Hüllenende 12 auf. Das offen ausgebildete distale Hüllenende 11 ist am proximalen Nadelträgerende 10 des Nadelträgers 2 befestigt. Dies kann durch einfaches Überschieben des offenen distalen Hüllenendes 11 über einen Ansatz mit einer Rückhaltevorrichtung am Nadelträger 2 erfolgen, wie dies allgemein und hinlänglich bekannt ist. Damit umschließt die Hülle 4 zumindest einen Teilabschnitt der Kanüle 3 im Bereich von deren proximalen Kanülenabschnitt 7 vollständig. Durch dieses vollvollständige Umschließen wird eine Aufnahmekammer 13 innerhalb der Hülle 4 ausgebildet, welche einerseits durch den Hülleninnraum sowie andererseits durch den Halteansatz des Nadelträgers 2 dichtend abgeschlossen ist.

Des Weiteren ist eine Entlüftungsvorrichtung 14 innerhalb des Nadelträgers 2 vorgesehen, durch welche die in der Kanüle 3 sowie die in der Aufnahmekammer 13 vorhandene Luft an die äußere Umgebung abströmen kann. Dazu ist weiters im Nadelträger 2 ein Abströmkanal 15 mit bevorzugt mehreren Teilabschnitten vorgesehen, wobei im vorliegenden Ausführungsbeispiel die Entlüftungsvorrichtung 14 einen Teilabschnitt des Abströmkanals 15 ausbildet.

Das freie, schräg angeschliffene proximale Ende der Kanüle 3 mündet mit ihrem proximalen Kanülenabschnitt 7 in die Aufnahmekammer 13 der Hülle 4 ein. Die Innenfläche der Hülle 4 ist mit radialer Distanz bezüglich der Außenseite der Kanüle 3 in Axialrichtung verlaufend bis hin zum distalen Hüllenende 11 im Verbindungsbereich mit dem Nadelträger 2 ausgebildet. Dadurch entsteht im günstigsten Fall bei keinem Rundumanliegen der Hülleninnenwand an der Außenseite der Kanüle 3 ein sich hin bis zum Nadelträger 2 erstreckender Ringkanal, welcher einen ersten Teilabschnitt des Abströmkanal 15 bildet.

Wie bereits zuvor beschrieben, kann der Nadelträger 2 durch eine Bauteilgruppe aus mehreren Bauteilen gebildet sein. Da es sich bei derartigen medizinischen Abnahmeeinheiten, welche mit Blut in Kontakt kommen, um Einwegprodukte für eine Einmalverwendung handelt, soll mit geringstem Aufwand an Bauteilen sowie Bauteilkosten trotzdem eine sichere Anwendung garantiert sein. Dies ist insbesondere zur Vermeidung von Ansteckungen oder Übertragung von Infektionen, wie beispielsweise AIDS, Hepatitis oder dergleichen ein wesentlicher Aspekt.

Im vorliegenden Ausführungsbeispiel umfasst der Nadelträger 2 einen Basiskörper 16, der an seinem proximalen Endbereich 17 einen sich in sein Inneres hinein erstreckenden Aufnahmeraum 18 aufweist. Der Aufnahmeraum 18 ist durch eine Innenfläche 19 begrenzt. Des Weiteren umfasst der Nadelträger 2 noch einen im Aufnahmeraum 18 aufgenommen und am Basiskörper 16 gehaltenen Einsatzteil 20, der seinerseits eine der Innenfläche 19 zugewendete Außenfläche 21 aufweist.

Ein zweiter Abschnitt des Abströmkanals 15 ist in Abströmrichtung anschließend an die Aufnahmekammer 13 sowie dem zwischen der Hülle 4 und der Kanüle 3 ausgebildeten Zwischenraum in weiterer Folge zwischen der Außenseite der Kanüle 3 und dem Einsatzteil 20 des Nadelträgers 2 verlaufend angeordnet oder ausgebildet. Bei dieser Ausbildung des Abströmkanals 15 ist beispielsweise vorgesehen, dass die Innenseite des Einsatzteils 20 über die gesamte Axialerstreckung in radialer Richtung von der Außenseite der Kanüle 3 distanziert angeordnet bzw. ausgebildet ist. Damit ergibt sich in diesem Abschnitt ebenfalls ein durchlaufend ausgebildeter Ringkanal, der ein Weiterströmen der Luft ausgehend vom proximalen Kanülenabschnitt 7 in den Nadelträger 2 hinein ermöglicht.

Unabhängig davon wäre es aber auch möglich, den Abströmkanal 15 im Bereich des Einsatzteils 20 durch mehrere über den Innenumfang des Einsatzteils 20 zueinander versetzt verlaufende Längsnuten auszubilden, um so eine zumindest teilweise Anlage bzw. Abstützung des Einsatzteils 20 an der Kanüle 3 zu ermöglichen. In diesem Fall kann noch vorgesehen sein, dass auch eine gewisse Halterung bzw. Befestigung des Einsatzteils 20 an der Kanüle 3 erfolgt. Dies könnte über einen Presssitz und/oder eine Klebeverbindung und/oder eine Schweißverbindung oder dergleichen erfolgen.

Des Weiteren kann innerhalb des Abströmkanals 15 eine Indikationskammer 22 zur Anzeige des Ansticherfolgs für die nachfolgende Blutabnahme vorgesehen bzw. ausgebildet sein. Die Indikationskammer 22 ist der Entlüftungsvorrichtung 14 in Strömungsrichtung der Luft gesehen vorgeordnet.

Der Einsatzteil 20 weist in Axialrichtung - also in Richtung der Längsachse 5 gesehen - ein distales Ende 23 sowie ein proximales Ende 24 auf. Dabei bildet das proximale Ende 24 des Einsatzteils 20 auch das proximale Nadelträgerende 10 aus.

Durch das Einsetzen des Einsatzteil 20 in den Aufnahmeraum 18 des Basiskörpers 16 kann die Indikationskammer 22 durch einen Teilabschnitt des Aufnahmeraums 18 des Basiskörpers 16 sowie dem distalen Ende 23 des Einsatzteils 20 ausgebildet bzw. begrenzt werden. Innerhalb des Abnahmekanals 8 zwischen dem distalen Kanülenabschnitt 6 sowie dem proximalen Kanülenabschnitt 7 ist eine erste Strömungsrichtung mit einem Pfeil 25 eingetragen. Diese erste Strömungsrichtung gemäß dem Pfeil 25 ist auch jene Strömungsrichtung, welche das abzunehmende Blut ausgehend von der Vene oder der Arterie des Patienten hin zum Blutentnahmeröhrchen nimmt. Durch das Eintreten von Körperflüssigkeit, insbesondere Blut, in den Abnahmekanal 8 wird die dort vorhandene Luft aus dem Abnahmekanal 8 hin zum proximalen Kanülenabschnitt 7 und weiter in die Aufnahmekammer 13 bewegt bzw. gedrückt.

Da der venöse Blutdruck üblicherweise in einem Bereich mit einer unteren Grenze von 3 mmHg (Millimeter-Quersilbersäule) und einer oberen Grenze von 8 mmHg liegt, ist es für eine selbsttätige Fließbewegung innerhalb des Abnahmekanals 8 notwendig, dass die dort vorhandene Luftmenge verdrängt wird. Der zuvor zwischen den Grenzen angegebene Druckbereich des Blutdruckes beträgt in einer anderen Maßeinheit z.B. 20 mbar (Millibar) bis 120 mbar. Da ein Komprimieren von Luft mit derartig geringen Drücken und dem geringen Platzangebot innerhalb des Nadelträgers 2 bzw. der Aufnahmekammer 13 nicht möglich ist, ist der zuvor beschriebene Abströmkanal 15 mit seinen unterschiedlichen Teilabschnitten, insbesondere der Entlüftungsvorrichtung 14 vorgesehen.

In diesem Abströmkanal 15 erfolgt beim vorliegenden Ausführungsbeispiel ausgehend von der Aufnahmekammer 13 durch den Einsatzteil 20 hindurch gegebenenfalls in die Indikationskammer 22 ein Abströmen der Luft in entgegengesetzter Strömungsrichtung, wie dies mit einem weiteren Pfeil 26 im Bereich der Außenseite der Hülle 4 angedeutet ist. Des Weiteren soll der Abströmkanal 15 so dimensioniert sein, dass bei unverformter Hülle 4 und erfolgtem Anstich des distalen Kanülenabschnitts 6 die vom Patienten abzunehmende Körperflüssigkeit, insbesondere das Blut, durch den Abnahmekanal 8 hindurch zumindest bis zum proximalen Kanülenabschnitt 7 in der ersten Strömungsrichtung gemäß Pfeil 25 in die Aufnahmekammer 13 einströmt. Ist beispielsweise die Hülle 4 selbst oder ein Teilbereich derselben aus einem transluzenten oder transparenten Werkstoff gebildet, kann hier bereits der Ansticherfolg optisch sichtbar gemacht werden. In diesem Fall könnte auf das Vorsehen bzw. das Anordnen der Indikationskammer 22 verzichtet werden.

Ist dies nicht der Fall, ist ein ungehindertes anschließendes Weiterströmen des eintretenden Blutes in der dazu entgegengesetzten Strömungsrichtung gemäß Pfeil 26 bis hin in die Indikationskammer 22 zu ermöglichen. Dabei sei erwähnt, dass die Indikationskammer 22 auch an einer Position innerhalb des Abströmkanals 15 angeordnet bzw. vorgesehen werden kann. Dazu ist es vorteilhaft wenn der Nadelträger 2, insbesondere dessen Basiskörper 16 zumindest bereichsweise aus einem transluzenten oder transparenten Werkstoff gebildet ist.

Die Kanüle 3 wird üblicherweise aus einem metallischen Werkstoff, insbesondere einem rostfreien, hochqualitativen Edelstahl - Werkstoff gebildet, um so einerseits die Einstichbelastung so gering wie möglich zu halten und andererseits ein Infektionsrisiko zu vermeiden.

Durch das bevorzugte Vorsehen einer durchgängigen, doppelendigen Kanüle 3 wird der Abnahmekanal 8 mit einem durchgängigen gleichen Strömungsquerschnitt ausgebildet. Dies hat den Vorteil, dass beim Hindurchströmen des Blutes bei der Abnahme keine negative Beeinflussung des Blutes, wie beispielsweise Hämolysen, oder dergleichen auftreten. Damit kann eine durch den Abnahmevorgang unbeeinflusste Probenqualität des Blutes beibehalten bzw. erreicht werden.

Die Entlüftungsvorrichtung 14, welche durch zumindest den im Nadelträger 2 angeordneten oder ausgebildeten weiteren Teilabschnitt des Abströmkanals 15 gebildet ist, erstreckt sich in weiterer Folge innerhalb des Nadelträgers 2 zwischen der den Aufnahmeraum 18 begrenzenden Innenfläche 19, sowie die den Basiskörper 16 ausbegrenzende Außenfläche 21. In diesem weiteren Strömungsabschnitt des Abströmkanals 15 erfolgt wiederum ein Richtungswechsel der Strömungsrichtung. Diese weitere Strömungsrichtung ist im vorliegenden Ausführungsbeispiel wiederum in gleicher Richtung ausgerichtet, wie dies bei der zuvor mit dem Pfeil 25 eingetragenen ersten Strömungsrichtung im Abnahmekanal 8 eingetragen ist. Damit ist die erste sowie letzte Strömungsrichtung von distal zu proximal und die zweite Strömungsrichtung von proximal zu distal verlaufend ausgerichtet.

So ist ein weiterer Teilabschnitt des Abströmkanals 15 durch mehrere in Ausströmrichtung nacheinander angeordnete, voneinander distanzierte Kammern 27, 28, 29 gebildet, wobei sich zwischen den voneinander distanzierten Kammern 27, 28, 29 zumindest ein sich zwischen unmittelbar in Ausströmrichtung benachbart angeordneten Kammern 27, 28; 28, 29 diese jeweils verbindender Verbindungskanal 30, 31 erstreckt. Diese bilden dann gemeinsam die Entlüftungsvorrichtung 14 aus.

Der oder die Verbindungskanäle 30, 31 weisen dabei einen Strömungsquerschnitt auf, welcher ein Hindurchströmen von Luft ermöglicht, jedoch ein Durchtreten der Körperflüssigkeit, insbesondere Blut zumindest zum überwiegenden Anteil verhindert oder diesen gänzlich unterbindet. Bevorzugt werden dabei jeweils in Strömungsrichtung unmittelbar nacheinander angeordnete Verbindungskanäle 30, 31 zwischen jeweils unmittelbar nacheinander angeordneten Kammern 27, 28, 29 jeweils in Umfangsrichtung zu einander versetzt angeordnet. Dabei kann die umfangsmäßige Versetzung z.B. diametral zueinander gewählt werden. Dabei sei erwähnt, dass die Anzahl sowohl der Kammern 27 bis 29 als auch der Verbindungskanäle 30, 31 frei gewählt werden kann. Im vorliegenden Ausführungsbeispiel sind drei Kammern 27 bis 29 gezeigt, wobei es aber möglich ist, eine dazu größere oder auch eine dazu geringere Anzahl zu wählen. Die einzelnen Kammern 27 bis 29 weisen bezüglich der Verbindungskanäle 30, 31 ein dazu wesentlich größeres Volumen bzw. einen vielfach größeren Strömungsquerschnitt auf. Dies deshalb, falls bei einem mehrfachen Abnahmevorgang jeweils mit der gleichen Abnahmevorrichtung 1 das am Beginn des Abströmkanals 15 vorhandene

Blut durch das mehrfache Einschieben und Anstechen des Blutprobenentnahmeröhrchens an die Kanüle 3 im Abströmkanal 15 unter Druck gesetzt werden kann. Durch diesen Druckaufbau und den dabei gegebenenfalls erzeugten Überdruck kann auch eine minimale Menge durch einen der Verbindungskanäle 30, 31 in Ausströmrichtung der Luft in eine der Kammern 27 bis 29 hineingedrückt werden.

Wie bereits zuvor beschrieben, sind die einzelnen Kammern 27 bis 29 zwischen der Innenfläche 19 des Aufnahmeraums 18 des Basiskörpers 16 und der Außenfläche 21 des Einsatzteils 20 angeordnet oder ausgebildet. Weiters ist bei diesem hier gezeigten Ausführungsbeispiel vorgesehen, dass im Axialschnitt gesehen die Innenfläche 19 des Aufnahmeraums 18 des Basiskörpers 16 in Richtung auf seinen proximalen Endbereich 17 hin stufenförmig erweiternd ausgebildet ist. Desgleichen ist im Axialschnitt gesehen aber auch eine Außenfläche 21 des Einsatzteils 20 in Richtung auf sein proximales Ende 24 stufenförmig erweiternd ausgebildet. Durch eine entsprechende maßliche Abstimmung zueinander kommt es so einerseits zu einer Ausbildung der einzelnen Kammern 27, 28, 29 sowie andererseits zu einem dichtenden Aneinanderliegen der Außenfläche 21 des Einsatzteils 20 an der Innenfläche 19 des Basiskörpers 16. Die Strömungsverbindung zwischen den damit ausgebildeten Kammern 27 bis 29 erfolgt durch die Verbindungskanäle 30, 31, welche bevorzugt vertieft in der Außenfläche 21 des Einsatzteils 20 angeordnet oder ausgebildet sind. So können die Verbindungskanäle 30, 31 beispielsweise durch Längsnuten mit einem sehr geringen Strömungsquerschnitt im Zusammenwirken mit der Innenfläche 19 des Aufnahmeraums 18 ausgebildet sein. Die Kammern 27 bis 29 können dabei jeweils über den Umfang durchlaufend ausgebildet sein. Bezogen auf die Längsachse 5 können diese eine kreisrunde Form aufweisen, wobei aber auch jegliche andere Form, wie oval, eckig, mehreckig oder polygonal möglich ist.

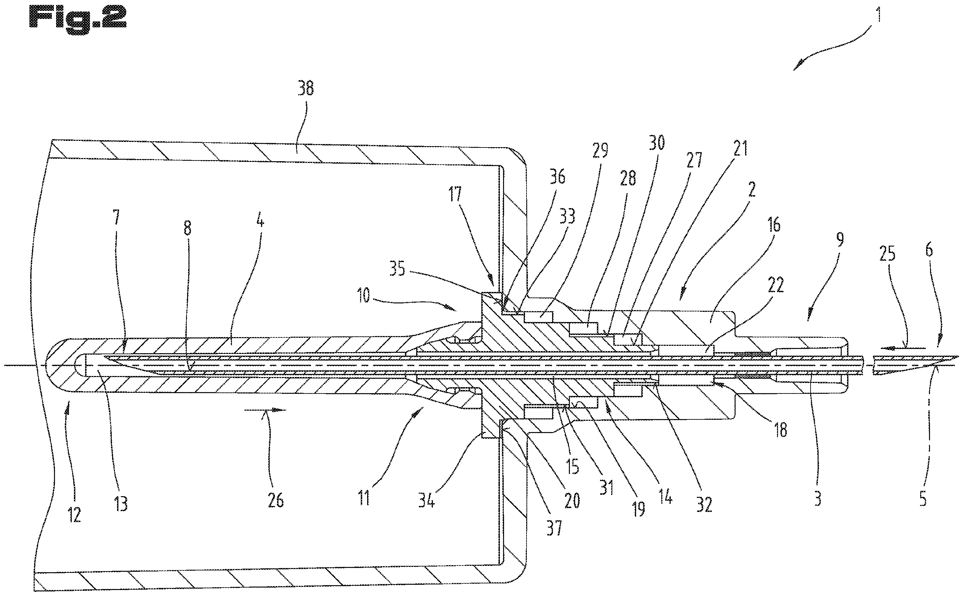

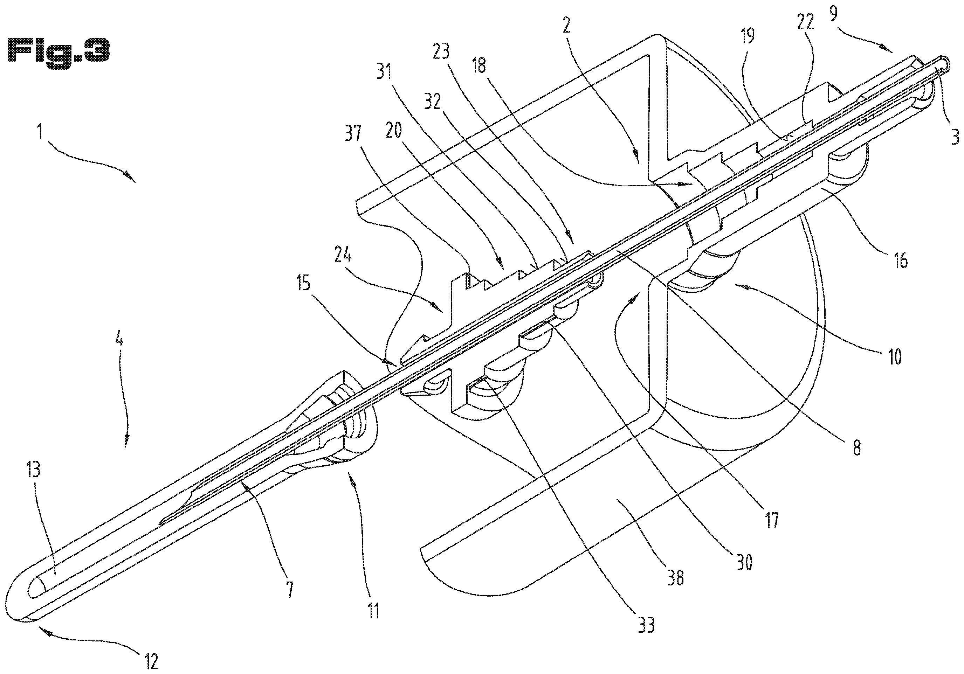

Um auch ein Hindurchströmen von Luft beispielsweise aus der Indikationskammer 22 des Abströmkanals 15 in die erste Kammer 27 zu ermöglichen, kann dazu ein erster weiterer Verbindungskanal 32 vorgesehen sein, welcher aus einer Zusammenschau der Fig. 2 und 3 zu ersehen ist. Das Ausströmen der Luft aus der hier in Strömungsrichtung letzten Kammer 29 kann durch einen zweiten weiteren Verbindungskanal 33 erfolgen. Dieser weitere Verbindungskanal 33 endet an einem im Bereich des proximalen Endes 24 des Einsatzteils 20 ausgebildet Ringansatz 34. Dieser flanschförmig ausgebildete Ringansatz 34 kann sich an einer Stirnwand 35 bzw. Stirnfläche des Basiskörpers 16 abstützen. Dabei können die aneinander zugewandten Flächen des Ringansatzes 34 sowie der Stirnwand 35 in einer senkrecht zur Längsachse 5 ausgerichteten Ebene verlaufend angeordnet sein. Durch das Abstützen des Ringansatzes 34 an der Stirnwand 35 kann sich zwischen dieser ausgehend vom weiteren Verbindungskanal 33 ein weiterer Abschnitt des Abströmkanals 15, nämlich ein Ringkanal 36 erstrecken. An einer dem weiteren Verbindungskanal 33 gegenüberliegenden Seite kann ein sich in radialer Richtung erstreckender Ausströmkanal 37 im Ringansatz 34 angeordnet bzw. ausgebildet sein. Über diesen Ausströmkanal 37 kann nun die durch den Abströmkanal 15 hindurchströmende Luft an die äußere Umgebung austreten.

Um eine Halterung der Kanüle 3 am Nadelträger 2 bzw. dessen Basiskörper 16 an der Kanüle 3 zu erzielen, ist bevorzugt der Basiskörper 16 feststehend mit der Kanüle 3 verbunden. Dies kann beispielsweise durch einen bekannten Klebevorgang erfolgen. Damit ist die Kanüle 3 in diesem Abschnitt dichtend mit dem Basiskörper 16 verbunden.

Der zuvor beschriebene Ringkanal 36 dient zum Abströmen der Luft aus dem Abströmkanal 15 kann durch entsprechende Freistellung von den einander jeweils zugewandten Eckbereichen zwischen dem Basiskörper 16 und dem Einsatzteil 20 erfolgen. Die einzelnen die Außenflächen 21 bildenden Stufen des Einsatzteils 20 sind hintereinander mit jeweils zunehmender radialer Distanz hin auf das proximale Ende 24 ausgebildet. Die Außenfläche 21 der letzten Stufe, an welche unmittelbar der Ringansatz 34 anschließt, kann in entsprechender Weise in ihren Eckbereich und damit den Übergang an den Übergang der Innenfläche 19 des Aufnahmeraums 18 an die Stirnwand 35 des Basiskörpers 16 erfolgen.

Der Durchströmquerschnitt jenes ersten weiteren Verbindungskanals 32, welcher hier im vorliegenden Ausführungsbeispiel die Indikationskammer 22 mit der ersten Kammer 27 sowie der die letzte Kammer 29 mit dem Ringkanal 36 verbindende zweite weitere Verbindungskanal 33 kann analog gewählt werden, wie dies bereits zuvor für die Verbindungskanäle 30, 31 beschrieben worden ist. Die zuvor beschriebene umfangsmäßige Versetzung der weiteren Verbindungskanäle 32, 33 bezüglich des ersten Verbindungskanals 30, welcher die erste Kammer 27 mit der zweiten Kammer 28 verbindet, sowie der zweite Verbindungskanal 31, welcher die zweite Kammer 28 und die dritte Kammer 29 miteinander verbindet, erfolgen.

Damit wird, falls die Indikationskammer 22 vorgesehen ist, das Ausströmen der Luft zwischen dem Basiskörper 16 und dem Einsatzteil 20 derart erfolgen, dass die Luft zuerst über den ersten weiteren Verbindungskanal 32 in die erste Kammer 27 einströmen kann. Das Weiterströmen der Luft aus der ersten Kammer 27 in die zweite Kammer 28 erfolgt über den ersten Verbindungskanal 30, welcher umfänglich versetzt, nämlich gegenüberliegend bezüglich des ersten weiteren Verbindungskanals 32, am Einsatzteil 20 als Längsnut ausgebildet ist. Die in die zweite Kammer 28 eingetretene Luft strömt über den zweiten Verbindungskanal 31 in die dritte Kammer 29 weiter, wobei dies wiederum auf der gegenüberliegenden Seite bezüglich des ersten Verbindungskanals 30 erfolgt. Aus der dritten und hier letzten Kammer 29 setzt sich der Abströmkanal 15 im zweiten weiteren Verbindungskanal 33 fort und mündet in den Ringkanal 36 ein. Das Ausströmen aus dem Ringkanal 36 erfolgt auf der gegenüberliegenden Seite bzgl. des zweiten weiteren Verbindungskanals 32 durch den sich in Radialrichtung erstreckenden Ausströmkanal 37 an die äußere Umgebung.

Durch das Vorsehen der einzelnen Verbindungskanäle 30 bis 33 mit dem sehr geringen Durchströmquerschnitt sowie die dazwischen liegenden Kammern 27 bis 29 mit einem dazu wesentlich größeren Durchströmquerschnitt bzw. Aufnahmevolumen kommt es hier zu einer Bildung einer sogenannten Labyrinthdichtung. Diese soll aufgrund der sehr gering gewählten Durchströmquerschnitte der Verbindungskanäle 30 bis 33 verhindern, dass in diesen Abschnitt des Abströmkanals 15 Blut eindringen kann. Sollte dies dennoch aus den zuvor beschriebenen Gründen bei mehreren aufeinanderfolgenden Abnahmevorgängen erfolgen, dienen die einzelnen Kammern 27 bis 29 als Speicherraum, um so sicher ein einen Blutaustritt aus dem Abströmkanal 15, insbesondere dessen Ausströmkanal 37 zu verhindern.

Um ein Austreten von Blut bzw. ein Einströmen desselben in die Kammern 27 bis 29 zu erschweren bzw. überhaupt zu verhindern bzw. dort eingetretenes Blut an einem Weiterströmen zu hindern, könnte in die Indikationskammer 22 und/oder zumindest eine der Kammern 27 bis 29 und/oder zumindest einen der Verbindungskanäle 30 bis 33 eine Substanz eingebracht werden, welche beispielsweise dazu dient, eingetretenes Blut gerinnen und somit stocken bzw. verfestigen zu lassen und damit eine gewisse Pfropfenwirkung durch verfestigtes Blut bzw. dessen Bestandteile zu erzielen. Damit kann eine noch bessere Abdichtung des Abströmkanals 15 in diesem Abschnitt erfolgen. Durch die Wahl der sehr geringen Strömungsquerschnitte im Bereich der Verbindungskanäle 30 bis 33 könnte so eine bewusste Verstopfung und ein damit verbundener Verschluss des Abströmkanals 15 in diesem Abschnitt erzielt werden. So könnte die Dichtheit und damit ein Ausströmen von Blut aus dem Abströmkanal 15 an die Umgebung zusätzlich verhindert werden. Damit wird ein Kontakt mit der abgenommenen Körperflüssigkeit verhindert und so das Ansteckungsrisiko ausgeschaltet.

Unabhängig davon wäre es aber auch möglich, in der Indikationskammer 22 einen Flüssigkeiten aufnehmenden, nicht näher bezeichneten Bauteil einzusetzen, welcher das bis dorthin eingetretene Blut aufnimmt, den Ansticherfolg somit optisch kennzeichnet, jedoch das Blut an einem Weiterströmen hindert. Ist nämlich das Blut bis in die Indikationskammer 22 eingetreten, ist bereits so viel Luft aus dem Abströmkanal 15 abgeströmt, dass ab diesem Zeitpunkt jener Abschnitt des Abströmkanals 15, welcher sich zwischen der Innenfläche 19 des Aufnahmeraums 18 sowie der Außenfläche 21 des Einsatzteils 20 erstreckt, für ein weiteres Abströmen nicht mehr unbedingt notwendig ist.

Die in dieser ersten Ausführungsform dargestellte und beschriebene Abnahmeeinheit 1 umfasst im vorliegenden Ausführungsbeispiel noch ein zusätzliches Handhabungselement 38, wobei der Nadelträger 2, insbesondere dessen Basiskörper 16 mit dessen distalen Stirnende verbunden ist. Dabei wird eine bevorzugt einstückige Ausbildung des Basiskörpers 16 und des Handhabungselements 38 gewählt. Damit ragt der von der Hülle 4 abgedeckte proximale Kanülenabschnitt 7 in das Handhabungselement 38 hinein, wie dies allgemein bekannt ist.

Diese gesamte Baueinheit aus dem Handhabungselement 38 sowie der Abnahmeeinheit 1 kann als Handhabungsbaugruppe bzw. Handhabungseinheit für medizinische Zwecke bezeichnet werden. Das Handhabungselement 38 umfasst in bekannter Weise einen Hauptkörperteil, der in Richtung der Längsachse 5 voneinander distanziert ein distales Ende sowie ein proximales Ende aufweist. Dabei ist das proximale Ende offen ausgebildet und dient zur Aufnahme zumindest eines Teils einer nicht näher bezeichneten und dargestellten Blutabnahmevorrichtung, nämlich bevorzugt einen evakuierten Aufnahmebehälter. Im Bereich des distalen Endes ist eine bevorzugt in einer senkrecht bzgl. der Längsachse 5 ausgebildeten Ebene ausgerichtete Behälterwand angeordnet, welche mit dem Basiskörper 16 bevorzugt zu einer Einheit verbunden ist.

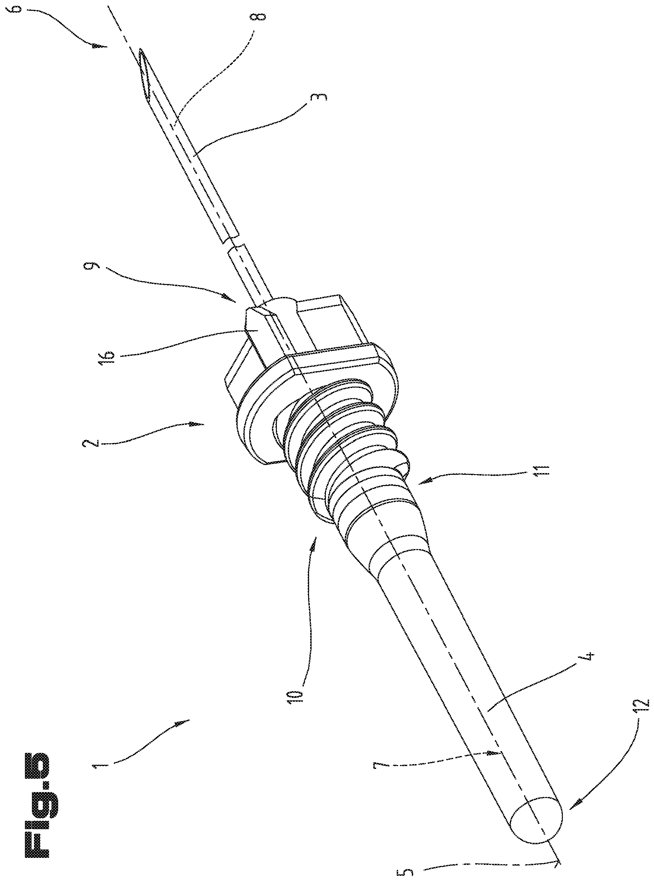

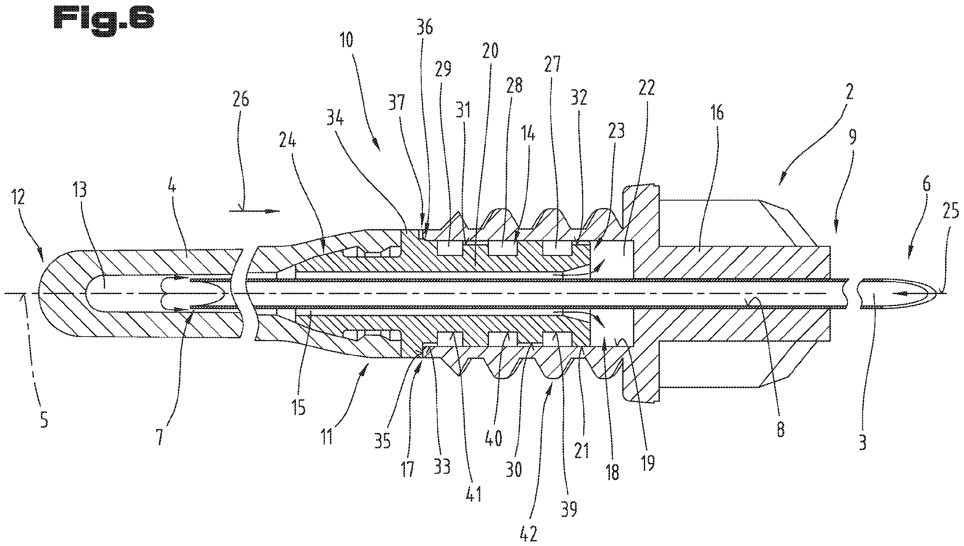

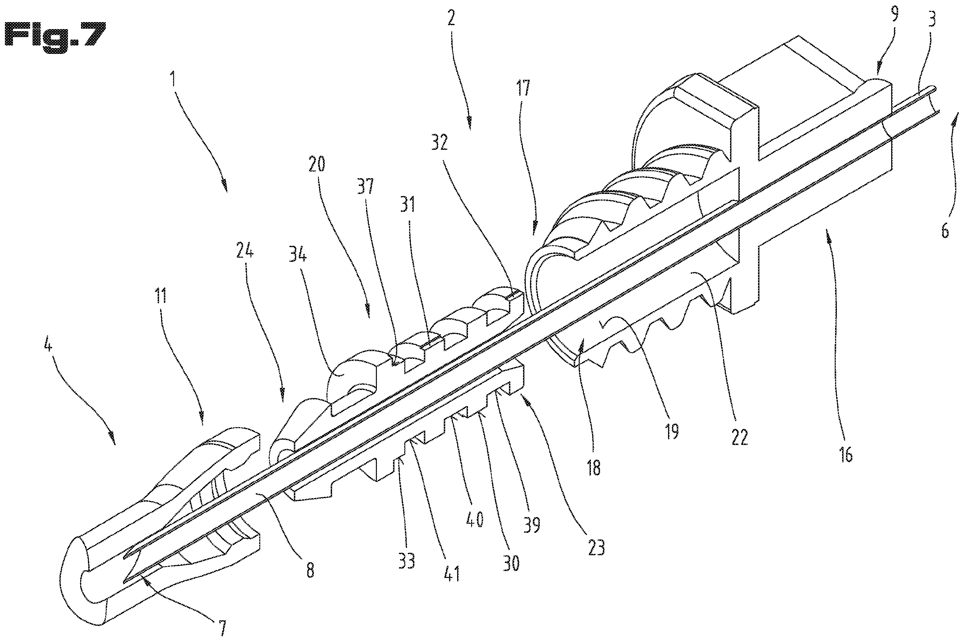

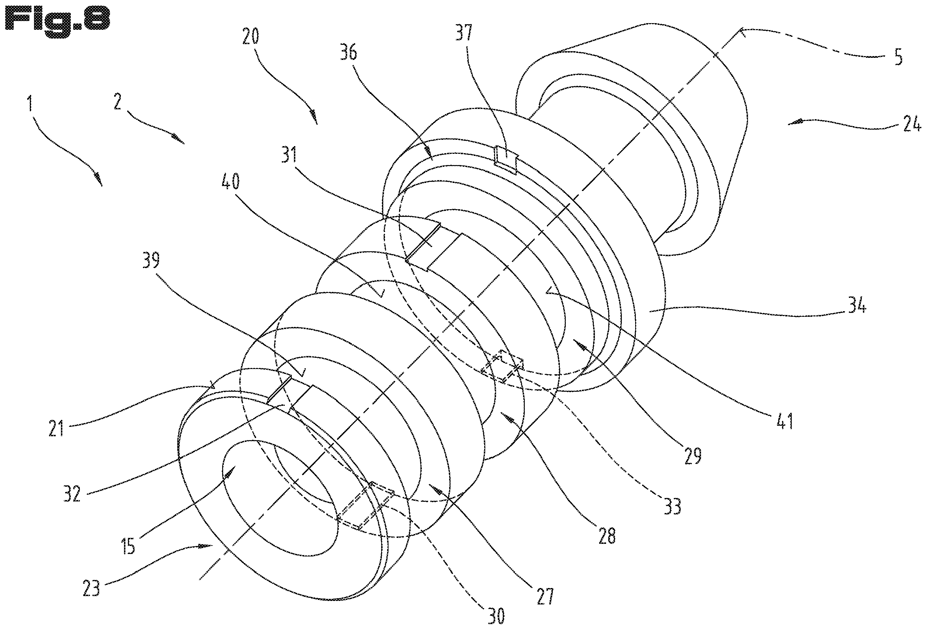

In den Fig. 5 bis 8 ist eine zweite und gegebenenfalls für sich eigenständige Ausführungsform der Abnahmeeinheit 1 gezeigt, wobei wiederum für gleiche Teile gleiche Bezugszeichen bzw. Bauteilbezeichnungen wie in den vorangegangenen Fig. 1 bis 4 verwendet werden. Um unnötige Wiederholungen zu vermeiden, wird auf die detaillierte Beschreibung in den vorangegangenen Fig. 1 bis 4 hingewiesen bzw. Bezug genommen.

Bei dieser hier gezeigten Ausbildung der Abnahmeeinheit 1 handelt es sich um eine vorgefertigte Baugruppe einer Kanülenanordnung, welche mit einem zuvor beschriebenen Handhabungselement 38 verbunden bzw. darin eingesetzt werden kann.

Die hier dargestellte Abnahmeeinheit 1 umfasst wiederum den aus dem Basiskörper 16 sowie dem Einsatzteil 20 gebildeten Nadelträger 2, der darin bzw. daran gehaltenen zumindest einen Kanüle 3, welche den proximalen Kanülenabschnitt 7 derselben abdeckt. Grundsätzlich entspricht der Aufbau, insbesondere des Nadelträgers 2 sowie der Hülle 4, jenem, wie dieser bereits detailliert in den Fig. 1 bis 4 beschrieben worden ist. Um unnötige Wiederholungen zu vermeiden, wird deshalb nur auf die Unterschiede zu den zuvor in den Fig. 1 bis 4 beschriebenen Ausführungsform detaillierter eingegangen.

Der Basiskörper 16 umfasst wiederum den Aufnahmeraum 18 mit seiner diesen begrenzenden Innenfläche 19. Im Gegensatz zu dem zuvor in den Fig. 1 bis 4 beschriebenen Aufnahmeraum 18 weist dieser Aufnahmeraum 18 eine in etwa zylindrische durchgehende, radial bezüglich der Längsachse 5 gleich distanzierte Innenfläche 19 auf. In diesen Aufnahmeraum 18 ist wiederum der Einsatzteil 20 eingesetzt, wobei auch hier die Außenfläche 21 zumindest in jenem, dem Aufnahmeraum 18 gegenüberliegenden Bereich ebenfalls zylindrisch bzgl. der Längsachse 5 ausgebildet ist. Zur Ausbildung der zuvor beschriebenen Kammern 27 bis 29 ist hier vorgesehen, dass diese jeweils durch in der Außenfläche 21 des Einsatzteils 20 vertieft ausgebildete Ausnehmungen 39 bis 41 gebildet sind. Je nach Anzahl der zu bildenden Kammern 27 bis 29 ist auch die entsprechende Anzahl der Ausnehmungen 39 bis 41 zu wählen. Wenn die Ausnehmungen 39 bis 41 zur Bildung der einzelnen voneinander getrennten Kammern 27 bis 29 über den Umfang durchlaufend ausgebildet sind, können diese als Nuten bzw. Ringnuten bezeichnet werden. Die einzelnen Ausnehmungen 39 bis 41 sind in Richtung der Längsachse 5 voneinander distanziert angeordnet und stehen jeweils über die zuvor beschriebenen Verbindungskanäle 30, 31 in Strömungsverbindung.

Der Abströmkanal 15 beginnt im Bereich der durch die Hülle 4 umschlossenen Aufnahmekammer 13 im Bereich des proximalen Kanülenabschnitts 7 und erstreckt sich auch hier zwischen der Außenseite der Kanüle 3 und der Innenseite des Einsatzteils 20 bis hin zur Indikationskammer 22, falls diese vorgesehen ist. Der weitere Strömungsweg führt über den ersten weiteren Verbindungskanal 32 in die erste Kammer 27, von dort über den ersten Verbindungskanal 30 in die zweite Kammer 28. Die zweite Kammer 28 ist über den zweiten Verbindungskanal 31 mit der dritten Kammer 29 verbunden. Schließlich steht die letzte und dritte Kammer 29 über den zweiten weiteren Verbindungskanal 33, den daran anschließenden Ringkanal 36 sowie den in radialer Richtung ausgerichteten Ausströmkanal 37 mit der äußeren Umgebung in Verbindung. Die zueinander umfangsmäßige Versetzung der einzelnen Verbindungskanäle 30 bis 33 kann analog erfolgen, so wie dies bereits zuvor detailliert beschrieben worden ist.

Zur Verbindung des Nadelträgers 2, insbesondere dessen Basiskörper 16, mit einem nicht näher dargestellten Handhabungselement 38 ist dieser mit einem Außengewinde 42 versehen. Damit kann der Nadelträger 2 mit einem nicht näher dargestellten distalen Stirnende eines üblichen Handhabungselements 38 bzw. Handhabungsvorrichtung zur Aufnahme eines Blutprobenröhrchens verbunden, insbesondere eingeschraubt werden. Bei dem Außengewinde 42 kann es sich um eine ein- oder mehrgängige Gewindeanordnung handeln, wobei dies in Abhängigkeit von dem jeweils damit zu verbindenden Handhabungselement 38 zu wählen ist.

Ist auch bei dieser Ausführung die Indikationskammer 22 vorgesehen, ist der Nadelträger 2, insbesondere dessen Basiskörper 16, zumindest bereichsweise aus einem transluzenten oder transparenten Werkstoff zu bilden. Es wäre aber wiederum auch möglich, dass die Hülle 4 zumindest bereichsweise aus einem transluzenten oder transparenten Werkstoff gebildet ist.

Der zuvor beschriebene Ringkanal 36 dient zum Abströmen der Luft aus dem Abströmkanal 15 und kann durch eine entsprechende Freistellung von den einander jeweils zugewandten Eckbereichen zwischen dem Basiskörper 16 und dem Einsatzteil 20 gebildet werden.

Der Durchströmquerschnitt jenes ersten weiteren Verbindungskanals 32, welcher hier im vorliegenden Ausführungsbeispiel die Indikationskammer 22 mit der ersten Kammer 27 sowie der die letzte Kammer 29 mit dem Ringkanal 36 verbindende zweite weitere Verbindungskanal 33 kann analog gewählt werden, wie dies bereits zuvor für die Verbindungskanäle 30, 31 beschrieben worden ist. Die zuvor beschriebene umfangsmäßige Versetzung der weiteren Verbindungskanäle 32, 33 bezüglich des ersten Verbindungskanals 30, welcher die erste Kammer 27 mit der zweiten Kammer 28 verbindet, und/oder des zweiten Verbindungskanals 31, welcher die zweite Kammer 28 und die dritte Kammer 29 miteinander verbindet, kann dazu analog erfolgen.

Damit wird, falls die Indikationskammer 22 vorgesehen ist, das Ausströmen der Luft zwischen dem Basiskörper 16 und dem Einsatzteil 20 derart erfolgen, dass die Luft zuerst über den ersten weiteren Verbindungskanal 32 in die erste Kammer 27 einströmen kann. Das Weiterströmen der Luft aus der ersten Kammer 27 in die zweite Kammer 28 erfolgt über den ersten Verbindungskanal 30, welcher umfänglich versetzt, nämlich gegenüberliegend bezüglich des ersten weiteren Verbindungskanals 32, am Einsatzteil 20 als Längsnut ausgebildet ist. Die in die zweite Kammer 28 eingetretene Luft strömt über den zweiten Verbindungskanal 31 in die dritte Kammer 29 weiter, wobei dies wiederum auf der gegenüberliegenden Seite bezüglich des ersten Verbindungskanals 30 erfolgt. Aus der dritten und hier letzten Kammer 29 setzt sich der Abströmkanal 15 im zweiten weiteren Verbindungskanal 33 fort und mündet in den Ringkanal 36 ein. Das Ausströmen aus dem Ringkanal 36 erfolgt auf der gegenüberliegenden Seite bzgl. des zweiten weiteren Verbindungskanals 32 durch den sich in Radialrichtung erstreckenden Ausströmkanal 37 an die äußere Umgebung.

Durch das Vorsehen der einzelnen Verbindungskanäle 30 bis 33 mit den jeweils sehr geringen Durchströmquerschnitten sowie der dazwischen liegenden Kammern 27 bis 29 mit einem dazu wesentlich größeren Durchströmquerschnitt bzw. Aufnahmevolumen kommt es hier zu einer Bildung einer sogenannten Labyrinthdichtung. Diese soll aufgrund der sehr gering gewählten Durchströmquerschnitte der Verbindungskanäle 30 bis 33 verhindern, dass in diesen Abschnitt des Abströmkanals 15 Blut eindringen kann. Sollte dies dennoch aus den zuvor beschriebenen Gründen bei mehreren aufeinanderfolgenden Abnahmevorgängen erfolgen, dienen die einzelnen Kammern 27 bis 29 als Speicherraum, um so sicher ein einen Blutaustritt aus dem Abströmkanal 15, insbesondere dessen Ausströmkanal 37 zu verhindern.

Um ein Austreten von Blut bzw. ein Einströmen desselben in die Kammern 27 bis 29 zu erschweren bzw. überhaupt zu verhindern bzw. dort eingetretenes Blut an einem Weiterströmen zu hindern, könnte in die Indikationskammer 22 und/oder zumindest eine der Kammern 27 bis 29 eine Substanz eingebracht werden, welche beispielsweise dazu dient, eingetretenes Blut gerinnen zu lassen und somit eine gewisse Pfropfenwirkung durch verfestigtes Blut bzw. dessen Bestandteile zu erzielen. Durch die Wahl der sehr geringen Strömungsquerschnitte im Bereich der Verbindungskanäle 30 bis 33 könnte so eine bewusste Verstopfung und ein damit verbundener Verschluss des Abströmkanals 15 in diesem Abschnitt erzielt werden. So könnte die Dichtheit und damit ein Ausströmen von Blut aus dem Abströmkanal 15 zusätzlich verhindert werden.

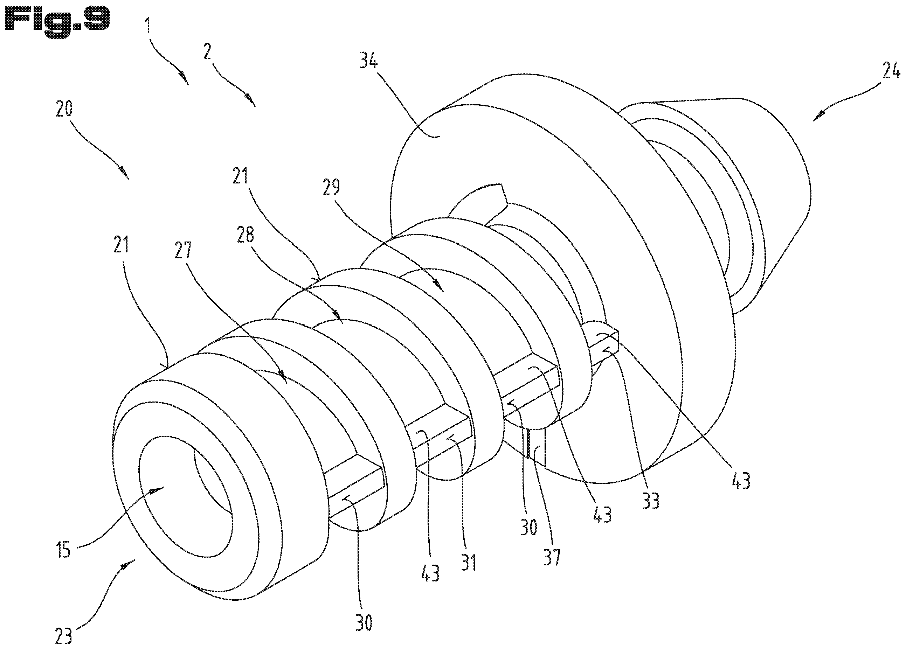

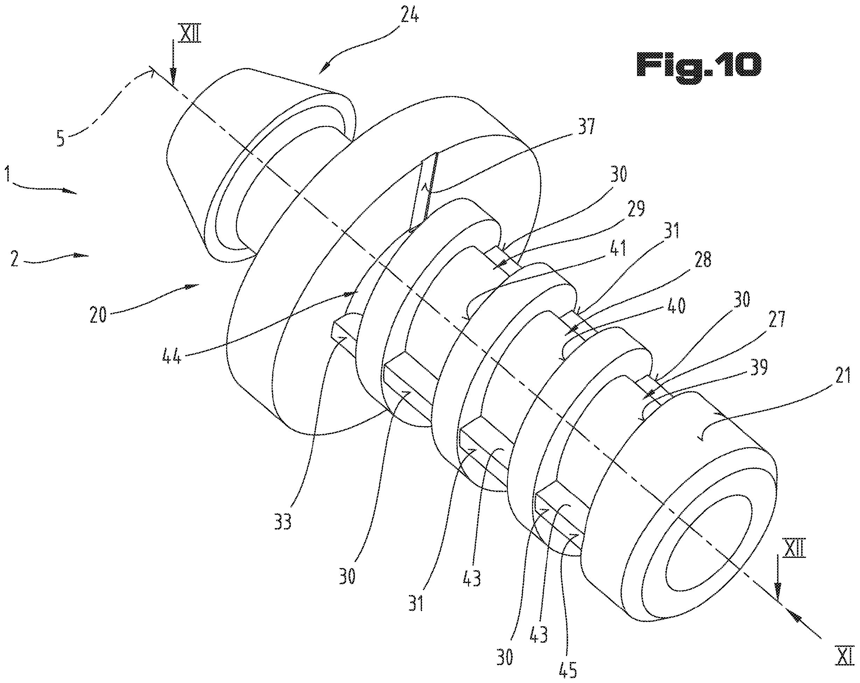

In den Fig. 9 bis 12 ist eine weitere und gegebenenfalls für sich eigenständige Ausführungsform der Abnahmeeinheit 1 gezeigt, wobei wiederum für gleiche Teile gleiche Bezugszeichen bzw. Bauteilbezeichnungen wie in den vorangegangenen Fig. 1 bis 8 verwendet werden. Um unnötige Wiederholungen zu vermeiden, wird auf die detaillierte Beschreibung in den vorangegangenen Fig. 1 bis 8 hingewiesen bzw. Bezug genommen.

Bei diesem hier gezeigten Ausführungsbeispiel ist lediglich der Einsatzteil 20 ohne den diesen aufnehmenden Basiskörper 16 sowie die Kanüle 3 dargestellt. Dieser weitere Einsatzteil 20 ist ähnlich ausgebildet, wie dieser zuvor in den Fig. 5 bis 8 beschrieben worden ist. Deshalb wird hier nur mehr auf die wesentlichsten Unterschiede detailliert eingegangen.

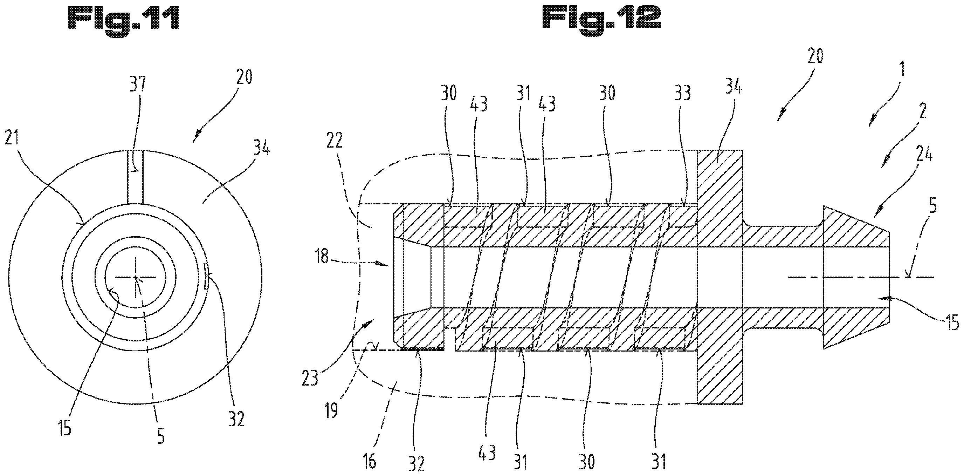

Der hier gezeigte Einsatzteil 20 wird wiederum in den Aufnahmeraum 18 des Basiskörpers 16 eingesetzt, wie dies schematisch in der Fig. 12 in strichlierten Linien angedeutet ist. Ein Teilabschnitt des Abströmkanals 15 zur Bildung der Entlüftungsvorrichtung 14 erstreckt sich innerhalb des Einsatzteils 20 ausgehend vom proximalen Ende 24 hin zum distalen Ende 23 und mündet dort, falls vorgesehen, in die Indikationskammer 22 ein. Diese wird zwischen dem distalen Ende 23 des Einsatzteils 20 und dem Aufnahmeraum 18 innerhalb des Basiskörpers 16 ausgebildet.

Anschließend erstreckt sich ein weiterer Abschnitt des Abströmkanals 15 im Bereich der Außenfläche 21 zwischen dem Einsatzteil 20 und dem Aufnahmeraum 18 mit seiner Innenfläche 19. Im Bereich der Außenfläche 21 des Einsatzteils 20 sind wiederum mehrere Kammern 27 bis 29 in Strömungsrichtung hintereinander angeordnet. Im Gegensatz zu der ringnutförmigen Ausbildung der einzelnen Ausnehmungen 39 bis 41, wie dies zuvor in den Fig. 5 bis 8 beschrieben worden ist, sind diese hier in Umfangsrichtung spiralförmig hintereinander angeordnet. In Umfangsrichtung gesehen sind die einzelnen Kammern 27 bis 29 bzw. die diese bildenden Ausnehmungen 39 bis 41 durch mehrere über den Umfang verteilt angeordnete Trennstege 43 zusätzlich begrenzt. Je nach gewählter Anzahl sowie Anordnung der Trennstege 43 kann damit die Anzahl der Kammern 27 bis 29 beliebig festgelegt werden. Bei diesem hier gezeigten Ausführungsbeispiel sind beispielsweise die Trennstege 43 diametral gegenüberliegend, also um 180° zueinander versetzt, angeordnet.

Der erste, weitere Verbindungskanal 32, welcher die Indikationskammer 22 mit der ersten Kammer 27 verbindet, ist bei diesem Ausführungsbeispiel in der gleichen Ebene angeordnet, in welcher auch die Trennstege 43 angeordnet sind. Je nach gewählter Steigung und der damit verbundenen Anzahl der Kammern 27 bis29 ist zwischen dem zweiten, weiteren Verbindungskanal 33 und dem Ausströmkanal 37 eine weitere Ausnehmung 44 in der Außenfläche 21 angeordnet, um so auch in diesem Bereich eine Strömungsverbindung auszubilden. Die einzelnen Trennstege 43 weisen bezüglich der Außenfläche 21 eine dazu geringere Distanz bezüglich der Längsachse 5 auf. Durch diese in radialer Richtung verkürzte Ausbildung der einzelnen Trennstege 43 wird zwischen einer äußeren Stirnfläche 45 der Trennstege 43 und der Innenfläche 19 des Aufnahmeraums 18 des Basiskörpers 16 jeweils der entsprechende Verbindungskanal 30 bzw. 31zwischen unmittelbar benachbart, hintereinander angeordneten Kammern 27 bis29 ausgebildet.

Im Gegensatz zu der hier zuvor beschriebenen und dargestellten Anordnungsmöglichkeit der Trennstege 43 wäre es aber auch möglich, sowohl deren Anzahl in Richtung der Längserstreckung der Ausnehmungen 39 bis 41 davon abweichend zu wählen. So könnte eine dazu höhere und/oder geringere Anzahl bezogen auf die Umfangslänge einer vollen Umfangserstreckung der jeweiligen Ausnehmung 39 bis 41 gewählt werden.

Dabei sei erwähnt, dass der Strömungsquerschnitt der einzelnen Verbindungskanäle 30 bis 33 zueinander ebenfalls unterschiedlich ausgebildet bzw. gewählt werden kann. So wäre es beispielsweise möglich, dass der Strömungsquerschnitt der einzelnen Verbindungskanäle 30 bis 33 ausgehend vom distalen Ende 23 des Einsatzteils 20 hin zum Ausströmkanal 37 stetig verkleinert ausgebildet wird. Damit kann eine noch bessere Flüssigkeitsabdichtung des Abströmkanals 15 in diesem Abschnitt des Einsatzteils 20 erzielt werden.

Der hier beschriebene Einsatzteil 20 kann in einen Basiskörper 16 mit oder ohne Außengewinde 42 eingesetzt werden, wie dieser zuvor in der ersten Ausführung gemäß der Fig. 1 bis 4 oder der zweiten Ausführung gemäß der Fig. 5 bis 8 beschrieben worden ist. Dabei ist in Abhängigkeit von der Außenform des Einsatzteils 20 die dazu entsprechende Raumform des Aufnahmeraums 18 zu wählen. Die zweite Ausführung gemäß der Fig. 5 bis 8 könnte aber auch bei entsprechender Ausbildung des Aufnahmeraums 18 in der ersten beschriebenen Ausführungsform mit dem Handhabungselement 38 eingesetzt werden.

Wesentlich ist bei all diesen Ausbildungen, dass stets auf den Einsatz von Filterelementen verzichtet wird und ausschließlich durch entsprechende Wahl, Größe, Anordnung sowie Ausbildung der Verbindungskanäle 30 bis 33 sowie der dazwischen angeordneten Kammern 27 bis 29 eine Abdichtung auf mechanischer Basis erfolgt. Damit können Kosten eingespart werden, da es sich bei derartigen Medizinprodukten stets um Einwegprodukte handelt, welche nach deren Verwendung zu entsorgen sind.

Wie bereits zuvor beschrieben, könnte innerhalb des Abströmkanals 15 zumindest in Teilabschnitten desselben eine Substanz oder ein Medium eingebracht bzw. darin aufgebracht werden, welche bzw. welches eine rasche Blutgerinnung bewirkt. Dies vor allem in jenen Abschnitten des Abströmkanals 15, um zuvor eine einwandfreie Anzeige des Ansticherfolgs durch das bei der Blutabnahme eintretende Blut sichtbar zu machen und bei Kontakt die Gerinnung zu bewirken und auch zu beschleunigen.

Unabhängig davon bzw. zusätzlich dazu wäre es aber auch noch möglich, z.B. den ersten weiteren Verbindungskanal 32, welcher die Indikationskammer 22 mit der ersten Kammer 27 verbindet, mit einem größeren Strömungsquerschnitt auszubilden als zuvor beschrieben. Damit wird die Möglichkeit geschaffen, dass die abgenommene Körperflüssigkeit, insbesondere das Blut, in die erste Kammer 27 des Abströmkanals 15 auch noch eintreten kann. Weiters kann dann in der hier ersten Kammer 27 eine eigenen Substanz bzw. Medium eingebracht bzw. darin aufgebracht sein, welches einen raschen Vortest bzw. Kurzanalyse der eingetretenen Körperflüssigkeit, insbesondere des Blutes durchführt und das Testergebnis z.B. optisch oder durch ein anderes Signal angezeigt wird. Damit kann je nach eingesetzter Substanz bzw. eingesetztem Medium infolge des angezeigten Testergebnisses die Bedienperson noch vor weiteren durchzuführenden Tätigkeiten entscheiden, ob eine Probe in ein Probenaufnahmegefäß eingefüllt werden soll oder nicht. Damit kann eine ansonsten nachfolgende Probenentnahme kurzfristig abgebrochen bzw. überhaupt nicht damit begonnen werden und es brauchen so keine Probenentnahmeröhrchen unnötig verschwendet werden.

Bei der optischen Anzeige kann z.B. ein Farbwechsel oder die Darstellung in einer von mehreren vorbestimmten Farben einen Rückschluss auf das Testergebnis zulassen. Kommt die mit der Substanz bzw. dem Medium eintretende Körperflüssigkeit, insbesondere Blut, in Kontakt damit, kann es zu der farblichen Anzeige oder Änderung der Ausgangsfarbe der Substanz bzw. des Mediums kommen. Es wären aber auch andere davon abweichende Anzeigemittel einsetzbar. Diese sollen nur ein rasches Vortestergebnis liefern, um so eine Entscheidungshilfe für den Beginn des Abnahmevorganges nach erfolgtem Anstich zu erhalten.

Das Verfahren zur Entnahme von Körperflüssigkeit, insbesondere von Blut, kann mit den unterschiedlichen Ausführungsformen der Abnahmeeinheit 1 wie folgt stets gleichartig durchgeführt werden.

Bei ordnungsgemäß, insbesondere bevorzugt steril, vorbereiteter Abnahmeeinheit 1 wird zuerst ein den distalen Kanülenabschnitt 6 abdeckendes Schutzelement, insbesondere eine Schutzkappe abgezogen, um so das dem Patienten zugewendete Einstichende der Kanüle 3 frei zu geben. Anschließend daran wird der distale Kanülenabschnitt 6 in den dafür vorgesehenen Körperteil eingestochen und so der Zugang zur abzunehmenden Körperflüssigkeit geschaffen. Durch diesen Anstichvorgang wird bei einem ordnungsgemäßen Ansticherfolg die abzunehmende Körperflüssigkeit, im vorliegenden Fall Blut, durch den Abnahmekanal 8 der Kanüle 3 bis hin zu ihrem proximalen Kanülenabschnitt 7 strömen. Bedingt durch dieses Einströmen wird die im Abnahmekanal 8 enthaltene Luft durch den Abströmkanal 15 hindurch an die äußere Umgebung aus diesem abgeführt bzw. abgeleitet. Dadurch, dass auch der proximale Kanülenabschnitt 7 durch die elastisch verformbare Hülle 4 vollständig abgedeckt ist, kann durch diese dicht ausgebildete Hülle 4 auch kein Abströmen von Luft erfolgen.

Ist bereits die Hülle 4 aus einem durchscheinenden bzw. transparenten Werkstoff gebildet, kann bereits in diesem Bereich der Ansticherfolg augenscheinlich durch das Bedienpersonal festgestellt werden. Sollte kein durchsichtiges Material verwendet werden, strömt die Körperflüssigkeit, nämlich das Blut, entlang der Außenseite der Kanüle 3 durch den dazwischen ausgebildeten Abströmkanal 15 bis hin zum Einsatzteil 20 und weiter durch diesen bis zur Indikationskammer 22. In dieser Indikationskammer 22 wird nunmehr der Ansticherfolg optisch sichtbar. Das Abströmen der Luft durch den weiteren daran anschließenden Abschnitt des Abströmkanals 15 erfolgt durch die zuvor beschriebenen Verbindungskanälen 30 bis 33 sowie die dazwischen angeordneten Kammern 27 bis 29. Die letzte Kammer 29 kann in weiterer Folge über den Ringkanal 36 sowie den Ausströmkanal 37 schließlich mit der äußeren Umgebung in Strömungsverbindung stehen. Bedingt durch die entsprechende Querschnittswahl der Verbindungskanälen 30 bis 33 sowie der dazwischen liegenden Kammern 27 bis 29 erfolgt in diesem Abschnitt des Abströmkanals 15 bevorzugt lediglich ein Abströmen von Luft, jedoch wird ein Durchtritt der Körperflüssigkeit, insbesondere Blut, im Bereich der Verbindungskanäle 30 bis 33 verhindert.

Dieses zuvor beschriebene Verfahren bzw. Vorgehen zur Abnahme von Körperflüssigkeiten, insbesondere Blut, kann gegebenenfalls für sich eine eigenständige Aufgabe der Erfindung darstellen und durch dieses erfindungsgemäße Vorgehen gelöst werden.

Die Ausführungsbeispiele zeigen mögliche Ausführungsvarianten der Abnahmeeinheit 1, wobei an dieser Stelle bemerkt sei, dass die Erfindung nicht auf die speziell dargestellten Ausführungsvarianten derselben eingeschränkt ist, sondern vielmehr auch diverse Kombinationen der einzelnen Ausführungsvarianten untereinander möglich sind und diese Variationsmöglichkeit aufgrund der Lehre zum technischen Handeln durch gegenständliche Erfindung im Können des auf diesem technischen Gebiet tätigen Fachmannes liegt.

Weiters können auch Einzelmerkmale oder Merkmalskombinationen aus den gezeigten und beschriebenen unterschiedlichen Ausführungsbeispielen für sich eigenständige, erfinderische oder erfindungsgemäße Lösungen darstellen.

Die den eigenständigen erfinderischen Lösungen zugrundeliegende Aufgabe kann der Beschreibung entnommen werden.

Sämtliche Angaben zu Wertebereichen in gegenständlicher Beschreibung sind so zu verstehen, dass diese beliebige und alle Teilbereiche daraus mitumfassen, z.B. ist die Angabe 1 bis 10 so zu verstehen, dass sämtliche Teilbereiche, ausgehend von der unteren Grenze 1 und der oberen Grenze 10 mit umfasst sind, d.h. sämtliche Teilbereiche beginnen mit einer unteren Grenze von 1 oder größer und enden bei einer oberen Grenze von 10 oder weniger, z.B. 1 bis 1,7, oder 3,2 bis 8,1, oder 5,5 bis 10.

Vor allem können die einzelnen in den Fig. 1 bis 4, 5 bis 8 sowie 9 bis 12 gezeigten Ausführungen den Gegenstand von eigenständigen, erfindungsgemäßen Lösungen bilden. Die diesbezüglichen, erfindungsgemäßen Aufgaben und Lösungen sind den Detailbeschreibungen dieser Figuren zu entnehmen.

Der Ordnung halber sei abschließend darauf hingewiesen, dass zum besseren Verständnis des Aufbaus der Abnahmeeinheit 1 diese bzw. deren Bestandteile teilweise unmaßstäblich und/oder vergrößert und/oder verkleinert dargestellt wurden.

**Bezugszeichenaufstellung**

| | | | |
|---|---|---|---|
| 1 | Abnahmeeinheit | 31 | Verbindungskanal |
| 2 | Nadelträger | 32 | Verbindungskanal |
| 3 | Kanüle | 33 | Verbindungskanal |
| 4 | Hülle | 34 | Ringansatz |
| 5 | Längsachse | 35 | Stirnwand |
| 6 | distaler Kanülenabschnitt | 36 | Ringkanal |
| 7 | proximaler Kanülenabschnitt | 37 | Ausströmkanal |
| 8 | Abnahmekanal | 38 | Handhabungselement |
| 9 | distales Nadelträgerende | 39 | Ausnehmung |
| 10 | proximales Nadelträgerende | 40 | Ausnehmung |
| 11 | distales Hüllenende | 41 | Ausnehmung |
| 12 | proximales Hüllenende | 42 | Außengewinde |
| 13 | Aufnahmekammer | 43 | Trennsteg |
| 14 | Entlüftungsvorrichtung | 44 | Ausnehmung |
| 15 | Abströmkanal | 45 | Stirnfläche |
| 16 | Basiskörper | | |
| 17 | proximaler Endbereich | | |
| 18 | Aufnahmeraum | | |
| 19 | Innenfläche | | |
| 20 | Einsatzteil | | |
| 21 | Außenfläche | | |
| 22 | Indikationskammer | | |
| 23 | distales Ende | | |
| 24 | proximales Ende | | |
| 25 | Pfeil | | |
| 26 | Pfeil | | |
| 27 | Kammer | | |
| 28 | Kammer | | |
| 29 | Kammer | | |
| 30 | Verbindungskanal | | |

## Patentansprüche

1. Medizinische Abnahmeeinheit (1) für Körperflüssigkeiten, insbesondere Blut, mit einem Nadelträger (2), der ein distales Nadelträgerende (9) sowie ein proximales Nadelträgerende (10) aufweist, zumindest einer Kanüle (3), die einen distalen Kanülenabschnitt (6) zum Einstechen in den Körper und einen proximalen Kanülenabschnitt (7) zum Durchstechen eines Verschlusskörpers eines Sammelbehälters aufweist, einem Abnahmekanal (8), der den distalen Kanülenabschnitt (6) mit dem proximalen Kanülenabschnitt (7) verbindet, einer schlauchförmigen elastisch verformbaren sowie durchstechbaren Hülle (4), die ein verschlossenes proximales Hüllenende (12) sowie ein offenes distales Hüllenende (11) aufweist, wobei die Hülle (4) mit dem distalen Hüllenende (11) am proximalen Nadelträgerende (10) des Nadelträgers (2) befestigt ist und den proximalen Kanülenabschnitt (7) abdichtend unter Ausbildung einer Aufnahmekammer (13) umschließt, und mit einer Entlüftungsvorrichtung (14), welche die Aufnahmekammer (13) zum Abströmen von Luft aus dieser mit der äußeren Umgebung verbindet, **dadurch gekennzeichnet, dass** die Entlüftungsvorrichtung (14) durch zumindest einen im Nadelträger (2) angeordneten oder ausgebildeten Abströmkanal (15) gebildet ist, wobei ein Teilabschnitt des Abströmkanals (15) mehrere in Ausströmrichtung nacheinander angeordnete, voneinander distanzierte Kammern (27, 28, 29) sowie jeweils zumindest einen, unmittelbar in Ausströmrichtung benachbart angeordnete Kammern (27, 28, 29) verbindenden Verbindungskanal (30, 31) umfasst und dass der Verbindungskanal (30, 31) einen Strömungsquerschnitt aufweist, welcher ein Hindurchströmen der Luft ermöglicht, jedoch ein Hindurchtreten der Körperflüssigkeit, insbesondere Blut, zumindest zum überwiegenden Anteil verhindert oder gänzlich unterbindet.

2. Abnahmeeinheit (1) nach Anspruch 1, **dadurch gekennzeichnet, dass** die Verbindungskanäle (30, 31) zwischen unmittelbar nacheinander angeordneten Kammern (27, 28, 29) jeweils in Umfangsrichtung zueinander versetzt, insbesondere diametral zueinander, angeordnet sind.

3. Abnahmeeinheit (1) nach Anspruch 1 oder 2, **dadurch gekennzeichnet, dass** ein durch jede der Kammern (27, 28, 29) gebildetes Aufnahmevolumen jeweils um ein vielfaches größer ausgebildet ist als jenes Volumen, das durch den Verbindungskanal (30, 31) ausgebildet ist.

4. Abnahmeeinheit (1) nach einem der vorhergehenden Ansprüche, **dadurch gekennzeichnet, dass** der Nadelträger (2) einen Basiskörper (16) mit einem an seinem proximalen Endbereich (17) ausgebildeten Aufnahmeraum (18) mit einer den Aufnahmeraum (18) begrenzenden Innenfläche (19) sowie einen im Aufnahmeraum (18) aufgenommen und am Basiskörper (16) gehaltenen Einsatzteil (20) mit einer der Innenfläche (19) zugewendeten Außenfläche (21) umfasst.

5. Abnahmeeinheit (1) nach Anspruch 4, **dadurch gekennzeichnet, dass** die Kammern (27, 28, 29) zwischen der Innenfläche (19) des Aufnahmeraums (18) des Basiskörpers (16) und der Außenfläche (21) des Einsatzteils (20) angeordnet oder ausgebildet sind.

6. Abnahmeeinheit (1) nach Anspruch 4 oder 5, **dadurch gekennzeichnet, dass** die Kammern (27, 28, 29) jeweils durch in der Außenfläche (21) des Einsatzteils (20) vertieft ausgebildete Ausnehmungen (39, 40, 41) gebildet sind.

7. Abnahmeeinheit (1) nach einem der Ansprüche 4 bis 6, **dadurch gekennzeichnet, dass** die die Kammern (27, 28, 29) bildenden Ausnehmungen (39, 40, 41) jeweils durch in Richtung einer Längsachse (5) voneinander distanziert angeordnet Ringnuten gebildet sind.

8. Abnahmeeinheit (1) nach einem der Ansprüche 4 bis 6, **dadurch gekennzeichnet, dass** die die Kammern (27, 28, 29) bildenden Ausnehmungen (39, 40, 41) in Umfangsrichtung spiralförmig hintereinander angeordnet und jeweils durch Trennstege (43) voneinander getrennt sind.

9. Abnahmeeinheit (1) nach Anspruch 4 oder 5, **dadurch gekennzeichnet, dass** im Axialschnitt gesehen die Innenfläche (19) des Aufnahmeraums (18) des Basiskörpers (16) in Richtung auf seinen proximalen Endbereich (17) stufenförmig erweiternd ausgebildet ist.

10. Abnahmeeinheit (1) nach einem der Ansprüche 4, 5 oder 9, **dadurch gekennzeichnet, dass** im Axialschnitt gesehen die Außenfläche (21) des Einsatzteils (20) in Richtung auf sein proximales Ende (24) stufenförmig erweiternd ausgebildet ist.

11. Abnahmeeinheit (1) nach einem der Ansprüche 1 bis 7, 9 oder 10, **dadurch gekennzeichnet, dass** die Kammern (27, 28, 29) jeweils über den Umfang durchlaufend ausgebildet sind.

12. Abnahmeeinheit (1) nach einem der vorhergehenden Ansprüche, **dadurch gekennzeichnet, dass** der Nadelträger (2), insbesondere dessen Basiskörper (16), mit der Kanüle (3) feststehend verbunden ist.

13. Abnahmeeinheit (1) nach einem der vorhergehenden Ansprüche, **dadurch gekennzeichnet, dass** der Abströmkanal (15) in Abströmrichtung unmittelbar anschließend an die Aufnahmekammer (13) zwischen der Kanüle (3) und dem Nadelträger (2), insbesondere dessen Einsatzteil (20), verlaufend angeordnet oder ausgebildet ist.

14. Abnahmeeinheit (1) nach einem der vorhergehenden Ansprüche, **dadurch gekennzeichnet, dass** innerhalb des Abströmkanals (15) eine Indikationskammer (22) zur Anzeige des Ansticherfolgs für die nachfolgende Blutentnahme ausgebildet ist.

15. Abnahmeeinheit (1) nach Anspruch 14, **dadurch gekennzeichnet, dass** die Indikationskammer (22) durch einen Teilabschnitt des Aufnahmeraums (18) des Basiskörpers (16) sowie einem distalen Ende (23) des Einsatzteils (20) ausgebildet ist.

16. Abnahmeeinheit (1) nach einem der vorhergehenden Ansprüche, **dadurch gekennzeichnet, dass** die Hülle (4) zumindest bereichsweise aus einem transluzenten oder transparenten Werkstoff gebildet ist.

17. Abnahmeeinheit (1) nach einem der vorhergehenden Ansprüche, **dadurch gekennzeichnet, dass** der Nadelträger (2), insbesondere dessen Basiskörper (16), zumindest bereichsweise aus einem transluzenten oder transparenten Werkstoff gebildet ist.

18. Abnahmeeinheit (1) nach einem der vorhergehenden Ansprüche, **dadurch gekennzeichnet, dass** der Abnahmekanal (8) durchgängig zwischen dem distalen Kanülenabschnitt (6) und dem proximalen Kanülenabschnitt (7) innerhalb der Kanüle (3) ausgebildet ist.

19. Abnahmeeinheit (1) nach einem der vorhergehenden Ansprüche, **dadurch gekennzeichnet, dass** der Nadelträger (2), insbesondere dessen Basiskörper (16), mit einem Außengewinde (42) versehen ist, wobei der Nadelträger (2) mit einem distalen Stirnende eines Handhabungselements (38) zur Aufnahme eines Blutabnahmeröhrchens verbindbar ist.

20. Abnahmeeinheit (1) nach einem der Ansprüche 1 bis 16, **dadurch gekennzeichnet, dass** der Nadelträger (2), insbesondere dessen Basiskörper (16), mit einem distalen Stirnende eines Handhabungselements (38) zur Aufnahme eines Blutabnahmeröhrchens verbunden ist.

## Claims

1. Medical removal unit (1) for bodily fluids, in particular blood, comprising a needle carrier (2), which needle carrier (2) has a distal needle carrier end (9) and a proximal needle carrier end (10), at least one cannula (3), which cannula (3) comprises a distal cannula section (6) for sticking into the body and a proximal cannula section (7) for piercing a closing body of a collecting container, a removal channel (8), which removal channel (8) connects the distal cannula section (6) to the proximal cannula section (7), a hose-like elastically deformable and pierceable sleeve (4), which sleeve (4) comprises a closed proximal sleeve end (12) and an open distal sleeve end (11), wherein the distal sleeve end (11) of the sleeve (4) is secured to the proximal needle carrier end (10) of the needle carrier (2) and surrounds the proximal cannula section (7) in a sealing manner forming an accommodating chamber (13), and with an air removal device (14), which air removal device (14) connects the accommodating chamber (13) for the outflow of air from the accommodating chamber (13) to the outer environment, wherein the air removal device (14) is formed by at least one outflow channel (15) arranged or formed in the needle carrier (2), wherein a portion of the outflow channel (15) comprises a plurality of chambers (27, 28, 29) arranged spaced apart from one another and behind one another in outflow direction and at least one connecting channel (30, 31) connecting chambers (27, 28, 29) arranged directly adjacent to one another in outflow direction and wherein the connecting channel (30, 31) has a flow cross-section which allows the through-flow of air, but prevents or completely blocks the passage of bodily fluid, in particular blood, at least for the most part.

2. The removal unit (1) as claimed in claim 1, wherein the connecting channels (30, 31) are arranged to be offset relative to one another in peripheral direction, in particular diametrically to one another, between chambers (27, 28, 29) arranged directly after one another.

3. The removal unit (1) as claimed in claim 1 or 2, wherein a receiving space formed by each of the chambers (27, 28, 29) is designed to be multiple times greater than the volume formed by the connecting channel (30, 31).

4. The removal unit (1) as claimed in any one of the preceding claims, wherein the needle carrier (2) comprises a main body (16) with a receiving space (18) formed at its proximal end section (17) with an inner surface (19) delimiting the receiving space (18) and an insert (20) received in the receiving space (18) and held on the main body (16) with an outer surface (21) facing the inner surface (19).

5. The removal unit (1) as claimed in claim 4, wherein the chambers (27, 28, 29) are arranged or formed between the inner surface (19) of the receiving space (18) of the main body (16) and the outer surface (21) of the insert (20).

6. The removal unit (1) as claimed in claim 4 or 5, wherein the chambers (27, 28, 29) are formed respectively by recesses (39, 40, 41) which are recessed in the outer surface (21) of the insert (20).

7. The removal unit (1) as claimed in any one of claims 4 to 6, wherein the recesses (39, 40, 41) forming the chambers (27, 28, 29) are formed respectively by annular grooves arranged spaced apart from one another in the direction of a longitudinal axis (5).

8. The removal unit (1) as claimed in any one of claims 4 to 6, wherein the recesses (39, 40, 41) forming the chambers (27, 28, 29) are arranged behind one another in circumferential direction and are separated from one another spiral-like respectively by separating webs (43).

9. The removal unit (1) as claimed in claim 4 or 5, wherein as viewed in axial cross-section the inner surface (19) of the receiving space (18) of the main body (16) is designed to widen step-like in the direction of its proximal end section (17).

10. The removal unit (1) as claimed in any one of claims 4, 5 or 9, wherein as viewed in axial cross-section the outer surface (21) of the insert (20) is designed to widen step-like in the direction of its proximal end (24).

11. The removal unit (1) as claimed in any one of claims 1 to 7, 9 or 10, wherein the chambers (27, 28, 29) are designed respectively to run continuously around the circumference.

12. The removal unit (1) as claimed in any one of the preceding claims, wherein the needle carrier (2), in particular its main body (16), is connected securely to the cannula (3).

13. The removal unit (1) as claimed in any one of the preceding claims, wherein the outflow channel (15) is arranged or designed to run in outflow direction immediately after the accommodating chamber (13) between the cannula (3) and the needle carrier (2), in particular its insert (20).

14. The removal unit (1) as claimed in any one of the preceding claims, wherein inside the outflow channel (15) an indication chamber (22) is provided for displaying the success of the penetration for the subsequent removal of blood.

15. The removal unit (1) as claimed in claim 14, wherein the indication chamber (22) is formed by a portion of the receiving space (18) of the main body (16) and a distal end (23) of the insert (20).

16. The removal unit (1) as claimed in any one of the preceding claims, wherein the sleeve (4) is made at least partly from a translucent or transparent material.

17. The removal unit (1) as claimed in any one of the preceding claims, wherein the needle carrier (2), in particular its main body (16), is made at least partly from a translucent or transparent material.

18. The removal unit (1) as claimed in any one of the preceding claims, wherein the removal channel (8) is designed to be continuous between the distal cannula (6) and the proximal cannula section (7) inside the cannula (3).

19. The removal unit (1) as claimed in any one of the preceding claims, wherein the needle carrier (2), in particular its main body (16), is provided with an external thread (42), wherein the needle carrier (2) can be connected to a distal face end of a handling element (38) for receiving a blood removal tube.

20. The removal unit (1) as claimed in any one of claims 1 to 16, wherein the needle carrier (2), in particular its main body (16), is connected to a distal face end of a handling element (38) for receiving a blood removal tube.

## Revendications

1. Unité de prélèvement médical (1) pour des fluides corporels, plus particulièrement du sang, avec un support d'aiguille (2), qui comprend une extrémité de support d'aiguille distale (9) ainsi qu'une extrémité de support d'aiguille proximale (10), au moins une canule (3), qui comprend une portion de canule distale (6) destinée à être piquée dans le corps et une portion de canule proximale (7) pour le perçage d'un corps de fermeture d'un récipient de collecte, un canal de prélèvement (8), qui relie la portion de canule distale (6) avec la portion de canule proximale (7), une enveloppe (4) en forme de gaine, déformable élastiquement et perforable, qui comprend une extrémité d'enveloppe proximale fermée (12) ainsi qu'une extrémité d'enveloppe distale ouverte (11), l'enveloppe (4) étant fixée, avec l'extrémité d'enveloppe distale (11), à l'extrémité de support d'aiguille proximale (10) du support d'aiguille (2) et entourant la portion de canule proximale (7) de manière étanche en formant une chambre de logement (13), et avec un dispositif de ventilation (14), qui relie la chambre de logement (13), pour l'évacuation de l'air hors de celle-ci, avec l'environnement extérieur, **caractérisée en ce que** le dispositif de ventilation (14) est constitué d'au moins un canal d'évacuation (15) disposé ou réalisé dans le support d'aiguille (2), une portion du canal d'évacuation (15) comprenant plusieurs chambres (27, 28, 29) disposées les unes après les autres dans la direction de l'évacuation et éloignées les unes des autres, ainsi qu'au moins un canal de liaison (30, 31) reliant les chambres (27, 28, 29) disposées de manière directement adjacente dans la direction de l'évacuation et **en ce que** le canal de liaison (30, 31) présente une section d'écoulement qui permet un passage de l'air mais qui empêche la pénétration du fluide corporel, plus particulièrement le sang, au moins en grande partie ou entièrement.

2. Unité de prélèvement (1) selon la revendication 1, **caractérisée en ce que** les canaux de liaison (30, 31) sont disposés entre des chambres (27, 28, 29) disposées de manière directement adjacente, de manière décalée entre eux dans la direction circonférentielle, plus particulièrement de manière diamétrale entre eux.

3. Unité de prélèvement (1) selon la revendication 1 ou 2, **caractérisée en ce qu'**un volume de logement formé par chacune des chambres (27, 28, 29) est conçu de façon à être plusieurs fois plus important que le volume formé par le canal de liaison (30, 31).

4. Unité de prélèvement (1) selon l'une des revendications précédentes, **caractérisée en ce que** le support d'aiguille (2) comprend un corps de base (16), avec un espace de logement (18) réalisé au niveau de sa partie d'extrémité proximale (17), avec une surface interne (19) limitant l'espace de logement (18) ainsi qu'un insert (20) logé dans l'espace de logement (18) et maintenu au niveau du corps de base (16), avec une surface externe (21) orientée vers la surface interne (19).

5. Unité de prélèvement (1) selon la revendication 4, **caractérisée en ce que** les chambres (27, 28, 29) sont disposées ou réalisées entre la surface interne (19) de l'espace de logement (18) du corps de base (16) et la surface externe (21) de l'insert (20).

6. Unité de prélèvement (1) selon la revendication 4 ou 5, **caractérisée en ce que** les chambres (27, 28, 29) sont formées chacune respectivement par des évidements (39, 40, 41) réalisées en creux dans la surface externe (21) de l'insert (20).

7. Unité de prélèvement (1) selon l'une des revendications 4 à 6, **caractérisée en ce que** les évidements (39, 40, 41) formant les chambres (27, 28, 29) sont formés chacun respectivement par des rainures annulaires disposées à une certaine distance entre elles en direction d'un axe longitudinal (5).

8. Unité de prélèvement (1) selon l'une des revendications 4 à 6, **caractérisée en ce que** les évidements (39, 40, 41) formant les chambres (27, 28, 29) sont disposés les uns derrière les autres en spirale dans la direction circonférentielle et sont séparées par des nervures de séparation (43).

9. Unité de prélèvement (1) selon la revendication 4 ou 5, **caractérisée en ce que**, en vue axiale, la surface interne (19) de l'espace de logement (18) du corps de base (16) est réalisée de façon à s'élargir par paliers en direction de sa partie d'extrémité proximale (17).

10. Unité de prélèvement (1) selon l'une des revendications 4, 5 ou 9, **caractérisée en ce que**, en vue axiale, la surface externe (21) de l'insert (20) est réalisée de façon à s'élargir par paliers en direction de son extrémité proximale (24).

11. Unité de prélèvement (1) selon l'une des revendications 1 à 7, 9 ou 10, **caractérisée en ce que** les chambres (27, 28, 29) sont réalisées chacune de manière continue sur la circonférence.

12. Unité de prélèvement (1) selon l'une des revendications précédentes, **caractérisée** en ce le support d'aiguille (2), plus particulièrement son corps de base (16), est relié de manière solidaire avec la canule (3).

13. Unité de prélèvement (1) selon l'une des revendications précédentes, **caractérisée en ce que** le canal d'évacuation (15) est disposé ou réalisé dans la direction d'évacuation de façon à s'étendre directement dans le prolongement de la chambre de logement (13) entre la canule (3) et le support d'aiguille (2), plus particulièrement son insert (20).

14. Unité de prélèvement (1) selon l'une des revendications précédentes, **caractérisée en ce que**, à l'intérieur du canal d'évacuation (15) est réalisée une chambre d'indication (22) pour l'affichage du succès du piquage pour le prélèvement sanguin suivant.

15. Unité de prélèvement (1) selon la revendication 14, **caractérisée en ce que** la chambre d'indication (22) est formée par une portion de l'espace de logement (18) du corps de base (16) ainsi que d'une extrémité distale (23) de l'insert (20).

16. Unité de prélèvement (1) selon l'une des revendications précédentes, **caractérisée en ce que** l'enveloppe (4) est constituée au moins partiellement d'un matériau translucide ou transparent.

17. Unité de prélèvement (1) selon l'une des revendications précédentes, **caractérisée en ce que** le support d'aiguille (2), plus particulièrement son corps de base (16), est constitué au moins partiellement d'un matériau translucide ou transparent.

18. Unité de prélèvement (1) selon l'une des revendications précédentes, **caractérisée en ce que** le canal de prélèvement (8) est réalisé de manière continue entre la portion de canule distale (6) et la portion de canule proximale (7) à l'intérieur de la canule (3).

19. Unité de prélèvement (1) selon l'une des revendications précédentes, **caractérisée en ce que** le support d'aiguille (2), plus particulièrement son corps de base (16), est muni d'un filetage externe (42), le support d'aiguille (2) pouvant être relié avec une extrémité frontale distale d'un élément de manipulation (38) pour le logement d'un tube de prélèvement de sang.

20. Unité de prélèvement (1) selon l'une des revendications 1 à 16, **caractérisée en ce que** le support d'aiguille (2), plus particulièrement son corps de base (16) est relié avec une extrémité frontale distale d'un élément de manipulation (38) pour le logement d'un tube de prélèvement sanguin.
